# FASCICULE DE BREVET EUROPEEN

(11) **EP 1 285 086 B1**
(45) Date de publication et mention de la délivrance du brevet: **03.07.2013**
(21) Numéro de dépôt: 01940647.9
(22) Date de dépôt: 30.05.2001
(51) Int. Cl.: C07D 407/12, C12Q 1/34, C12Q 1/26, C12Q 1/37, C07D 311/16

(54) **MÉTHODE DE LIBÉRATION D'UN PRODUIT COMPRENANT UNE OXYDATION CHIMIQUE, MÉTHODE DE DÉTECTION DUDIT PRODUIT ET LEURS APPLICATIONS**
OXIDATIVE METHODE DER FREISETZUNG EINER NACHWEISBAREN VERBINDUNG UND DEREN NACHWEIS
METHOD FOR LIBERATING A PRODUCT COMPRISING A CHEMICAL OXYDATION, DETECTION OF THE PRODUCT AND FURTHER APPLICATIONS

(30) Priorité: 30.05.2000 FR 0006952; 20.10.2000 FR 0013487
(43) Date de publication de la demande: 26.02.2003
(73) Titulaire: Proteus, 30000 Nîmes (FR); UNIVERSITE DE BERNE, CH-3010 Berne (CH)
(72) Inventeur: REYMOND, Jean-Louis, CH-3014 Berne (CH); WAHLER, Denis, CH-3012 Berne (CH); BADALASSI, Fabrizzio, I-57029 Venturina (IT); NGUYEN, Hong-Khanh, F-30000 Nimes (FR)
(74) Mandataire: Novagraaf Technologies
(86) Numéro de dépôt international: PCT/FR2001/001686
(87) Numéro de publication internationale: WO 2001/092563

(56) Documents cités:
- EP-A- 0 317 243
- EP-A- 0 796 854
- CHANG, GU GANG ET AL: "Modification of human placental alkaline phosphatase by periodate -oxidized 1,N6-ethenoadenosine monophosphate" BIOCHEM. J. (1990), 272(3), 683-90 , XP001034700
- HANESSIAN, STEPHEN ET AL: "Aminoglycoside antibiotics: chemical conversion of neomycin B, paromomycin, and lividomycin B into bioactive pseudosaccharides" CAN. J. CHEM. (1978), 56(11), 1482-91 , XP001041390
- LATIP JALIFAH ET AL: "Lignans and coumarins metabolites from Melicope hayesii." PHYTOCHEMISTRY (OXFORD), vol. 51, no. 1, mai 1999 (1999-05), pages 107-110, XP000986508 ISSN: 0031-9422
- MATSUMOTO TAKASHI ET AL: "Lipase-catalyzed hydrolysis of some racemic 1-acetoxy-2-arylpropanes." CHEMICAL & PHARMACEUTICAL BULLETIN (TOKYO), vol. 42, no. 6, 1994, pages 1191-1197, XP002160617 ISSN: 0009-2363
- M T REETZ, G LOHMER, R SCHWICKARDI: "A New Catalyst System for the Heck Reaction of Unreactive Aryl Halides" ANGEWANDTE CHEMIE. INTERNATIONAL EDITION, vol. 37, no. 4, 1998, pages 481-483, XP002160618
- JOURDAIN N ET AL: "A Stereoselective Fluorogenic Assay for Aldolases: Detection of an Anti-Selective Aldolase Catalytic Antibody" TETRAHEDRON LETTERS,NL,ELSEVIER SCIENCE PUBLISHERS, AMSTERDAM, vol. 39, no. 51, 17 décembre 1998 (1998-12-17), pages 9415-9418, XP004144215 ISSN: 0040-4039 cité dans la demande
- G KLEIN, J-L REYMOND: "Enantioselective Fluorogenic Assay of Acetate Hydrolysis for Detecting Lipase Catalytic Antibodies" HELVETICA CHIMICA ACTA, vol. 82, 1999, pages 400-407, XP002160619 cité dans la demande
- KLEIN G ET AL: "An Enantioselective Fluorimetric Assay for Alcohol Dehydrogenases Using Albumin-Catalyzed beta-Elimination of Umbelliferone" BIOORGANIC & MEDICINAL CHEMISTRY LETTERS,GB,OXFORD, vol. 8, no. 9, 1 mai 1998 (1998-05-01), pages 1113-1116, XP004137030 ISSN: 0960-894X cité dans la demande
- C A ROESCHLAUB, N L MAIDWELL, M REZA REZAI, P G SAMMES: "A fluorescent probe for the detection of NAD(P)H" CHEMICAL COMMUNICATIONS, 1999, pages 1637-1638, XP002160620 cité dans la demande
- LIST BENJAMIN ET AL: "Aldol sensors for the rapid generation of tunable fluorescence by antibody catalysis." PROCEEDINGS OF THE NATIONAL ACADEMY OF SCIENCES OF THE UNITED STATES, vol. 95, no. 26, 22 décembre 1998 (1998-12-22), pages 15351-15355, XP002160621 Dec. 22, 1998 ISSN: 0027-8424 cité dans la demande
- F BADALASSI, D WAHLER, G KLEIN, P CROTTI, J-L REYMOND: "A Versatile Periodate-Coupled Fluorogenic Assay for Hydrolytic Enzymes" ANGEWANDTE CHEMIE. INTERNATIONAL EDITION, vol. 39, no. 22, 17 novembre 2000 (2000-11-17), pages 4067-4070, XP002183671
- CHAI W ET AL: "SPECIFICITY OF MILD PERIODATE OXIDATION OF OLIGOSACCHARIDE-ALDITOLS: RELEVANCE TO THE ANALYSIS OF THE CORE-BRANCHING PATTERN OF O-LINKED GLYCOPROTEIN OLIGOSACCHARIDES", CARBOHYDRATE RESEARCH, PERGAMON, GB, vol. 239, 1 January 1993 (1993-01-01), pages 107-115, XP009021637, ISSN: 0008-6215, DOI: 10.1016/0008-6215(93)84207-M
- JEANES A ET AL: "PREPARATION OF meso-ERYTHRITOL AND d-ERYTHRONIC LACTONE FROM PERIODATE-OXIDIZED STARCH", JOURNAL OF ORGANIC CHEMISTRY, AMERICAN CHEMICAL SOCIETY, EASTON.; US, vol. 20, 1 January 1955 (1955-01-01), pages 1565-1568, XP002134519, ISSN: 0022-3263, DOI: 10.1021/JO01128A015
- Wagner And Zook: "Synthetic orgnaic chemistry", 1956, Joh Wiley & Sons Inc, New York pages 290-291,
- PENNINGTON ET AL: "Crystal structures of D-(+)- and meso-hydrobenzoin. Absolute direction of the dipole moment of D- and L-hydrobenzoin in the crystal and correlation with crystal morphology, pyroelectric effect, and absolute configuration", J. AM. CHEM. SOC., vol. 110, 1988, pages 6498-6504,

## Description

La présente invention a pour objet une méthode de détection de la transformation chimique et/ou enzymatique d'un substrat en un composé détectable, caractérisée en ce qu'elle comprend les étapes suivantes :
a) la transformation chimique d'un substrat de formule (I) dans laquelle la liaison C₁-C₂ est insensible à une coupure par une réaction d'oxydation chimique : 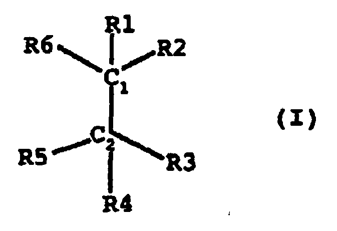 en un composé de formule (II) dans laquelle la liaison C₁-C₂ est sensible à une coupure par une réaction d'oxydation chimique effectuée au moyen d'un agent chimique oxydant : 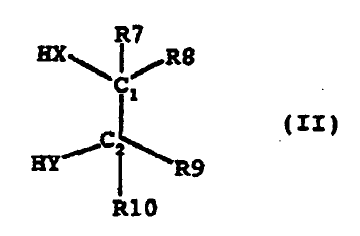 et,
(b) l'oxydation chimique du composé de formule (II) obtenu à l'étape (a) qui coupe la liaison C₁-C₂ pour obtenir directement ou indirectement un produit détectable,
et en ce que dans les composés de formules (I) et (II) :
- au moins un des groupes R₁ à R₆ est un groupement Di qui est insensible à la réaction d'oxydation de l'étape(b) et qui présente des propriétés détectables après la coupure de la liaison C₁-C₂,
- les groupes R₁ à R₆, identiques ou différents, sont choisis parmi un atome d'hydrogène, un groupe alkyle substitué ou non, un groupe fonctionnel substitué ou non et dans le cas d'un groupe fonctionnel de formules -OR₁₂, -SR₁₂, -NR₁₁R₁₂, R₁₁ est choisi parmi un atome d'hydrogène, un groupe alkyle, un groupe aryle, substitués ou non, et R₁₂ n'est pas un atome d'hydrogène,
- le ou les groupes R₁ à R₆ formant après l'étape (a) les groupes de formules -XH et -YH sont insensibles à l'oxydation de l'étape (b),
- X et Y, identiques ou différents, sont choisis parmi un atome d'oxygène, un atome de soufre, une amine de formule -NR₁₁R₁₂, R₁₁ est choisi parmi un atome d'hydrogène, un groupe alkyle, un groupe aryle, substitués ou non, et R₁₂ n'est pas un atome d'hydrogène,
- R₇ à R₁₀, identiques ou différents, sont, soit identiques à quatre au plus des groupes R₁ à R₆, soit, du fait de la transformation d'un ou plusieurs des groupes R₁ à R₆, lors de la réaction de l'étape (a), choisis parmi un atome d'hydrogène, un groupe alkyle substitué ou non, ou un groupe fonctionnel substitué ou non, et au moins un des groupes R₇ à R₁₀ est un groupement Di.

Chang et al (1990, Biochem J, 272, 683-690) décrivent l'oxydation du cis-diol du ribose présent sur le 1, N⁶-ethenoadenosine monophosphate (∈AMP) par le periodate pour conduire au dérivé dialdéhyde (∈AMP-dial) qui est un inhibiteur de la phosphatase alcaline du placenta humain utilisé comme marqueur d'affinité.

Hanessian et al (1977, Can. J Chem, 56(11), 1482-1491) décrivent la préparation d'antibiotiques aminoglycosidiques à partir de substances parentes dont les diols vicinaux ont subits une oxydation par le periodate suivi d'une β-élimination.

EP0796854 décrit un composé dérivé du benzopyrane et son utilisation pour traitrer des maladies cardiaques. Parmi ces dérivés du benzopyrane se trouvent les composés 3-ethoxy-4-hydroxy-7-(3,4-dihydroxybutoxy)-2H-1-benzopyranon-2-one, 3-butoxy-4-hydroxy-7-(3,4-dihydroxybutoxy)-2H-1-benzopyranon-2-one, 3-hexykixy-4-hydroxy-7-(3,4-dihydroxybutoxy)-2H-1-benzopyranon-2-one et 3-octyloxy-4-hydroxy-7-(3,4-dihydroxybutoxy)-2H-1-benzopyranon-2-one, qui sont tous des diols.

Laptip et al. (1999, Phytochemistry, 51(1), 107-110) décrivent un composé (7-(3-méthyl-4-carboxybutanoxy) umbellif érone méthyl ester) qui est un dérivé de la coumarine.

Matsumoto et al (1994, Chem Pharm Bull, 42(6), 1191-1197) décrivent une réaction d'hydrolyse de dérivés 1-acetoxy-2-arykpropanes racémiques par l'intermédiaire d'une lipase.

Reetz et al (1998, Angewandte Chemie, 37(4), 481-483) décrivent un système catalytique pour la réaction de Heck d'halogénures d'alkyle non réactifs, ainsi que le composé 2-ethylène-6-méthoxy naphtalène.

Jourdain et al (1998, Tetrahedron Letters, 39, 9415-9418) décrivent un test fluorogénique pour la détermination d'activités aldolases stéréosélectives utilisant un anticorps catalitique antialdolase pour bloquer sélectivement l'activité diastéreosélective des aldolases.

Klein et Reymond (1999, Helvetica Chimica Acta, 82, 400-407) décrivent une méthode de détection d'estérases ou de lipase au moyen de substrat pour obtenir des alcools lesquels sont transformés par une enzyme alcool déhydrogénase pour obtenir de l'umbelliférone.

Klein et Reymond (1998, Biorg Med Chem Lett, 8, 1113-1116) décrivent une méthode fluorométrique énantioséléctive pour la détection d'alcool déhydrogénases.

EP0317243 décrit une méthode de détermination de l'activité d'enzymes en permettant à un substrat formant une pigmentation détectable.

Roeschlaubet al (1999, Chem Communications, 1637-1638) décrivent une sonde fluorescente pour la détection du NAD(P)H.

List et al (1998, Proccedings of the Nat Ac of the Sc of the US, 95(26), 15351-15355), décrivent des susbstrats fluorogénique à base d'aldol pour la détection d'anticorps ayant une activité anti-aldolase.

Est également décrit dans la présente une méthode de libération d'au moins un produit pouvant être détecté, caractérisée en ce que l'on soumet un composé de formule (II') suivante : 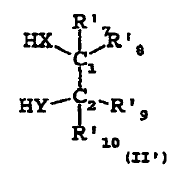
- à une oxydation chimique qui coupe la liaison C1-C2 pour obtenir ledit produit,
dans ledit composé de formule (II'):
- R'₇ à R'₁₀, identiques ou différents, correspondent à un atome d'hydrogène, un groupe alkyle substitué ou non, un groupe fonctionnel substitué ou non.
- X et Y, identiques ou différents, sont choisis parmi un atome d'oxygène, un atome de soufre, une amine de formule -NR₁₁R₁₂, R₁₁ est choisi parmi: un atome d'hydrogène, un groupe alkyle, un groupe aryle, substitués ou non, et R₁₂ n'est pas un atome d'hydrogène.

Selon l'invention la réaction d'oxydation chimique est réalisée au moyen d'un agent chimique oxydant pouvant correspondre de manière avantageuse et de façon non limitative à un ou plusieurs des réactifs suivants H₅IO₆, RuO₂, OsO₄, (CH₃CH₂CH₂)₄N(RuO₄), NaClO₄, NaIO₄, Na₃H₂IO₆, NaMnO₄, K₂OSO₄, KIO₄, KMnO₄, KRuO₄, K₂RuO₄, LiOCl, l'acétate de plomb, le tétrapropyle ammonium periodate, l'acide chromique ou des sels de celui-ci, NaBiO3, Ph3BiCO3, Ca(OCl)₂, les réactifs Ce(IV), Cr(VI), les sels de Co (II), IOAc, I(OAc)3, N-iodosuccinimide, VO(acac), Pb(OAc)₄, MnO₂, H₂O₂ ou le mélange des réactifs [H₂O₂, Na₂WO₄, H₃PO₄].

Tout préférentiellement l'agent chimique oxydant est un sel de périodate.

Ledit produit, directement ou indirectement libéré, peut être un molécule volatile ou une substance active capable de moduler une fonction telle que notamment une substance pharmacologique ou encore un composé détectable.

Dans la présente, on entend par :
- Molécule volatile : une ou plusieurs molécules seules ou en combinaison qui ont la propriété d'être volatiles, notamment toute molécule de type aldéhyde ou cétone volatile comme par exemple de nombreux parfums ou de nombreuses molécules bioactives telles que les phéromones.
- Substance active : une ou plusieurs molécules seules ou en combinaison capables de moduler une fonction telle que notamment une substance pharmaceutique capable par exemple de moduler une action hormonale, une action physiologique (la pression sanguine, humeur, état de veille), un développement malin (tumeur). Les substances actives peuvent également correspondre à des molécules ayant des propriétés curatives ou préventives d'une pathologie. Les substances actives peuvent encore avoir un effet antibiotique ou insecticide ou présenter des propriétés spécifiques d'odeur ou de goût.
- Composé spécifique: un ou plusieurs composés susceptibles de présenter seuls ou en combinaison une variation de leurs propriétés biophysiques, biologiques ou chimiques avant et après l'étape d'oxydation chimique, telles que notamment une variation de type spectral ou une variation de la solubilité.

- Oxydation chimique : toute réaction chimique réalisée en présence d'un réactif chimique susceptible d'oxyder le produit de formule (II') et induisant une coupure de la liaison entre les carbones C1 et C2.
- Groupe fonctionnel : tout groupe chimique appartenant à une classe de composés organiques caractérisée par des propriétés chimiques. On peut citer à titre d'exemple de groupe fonctionnel : des amides, des acyles, des alcoxy, des nitriles, des aryles, des hétéroaryles, des alkenyles, des carbonyles, des thiocarbonyles, des carboxyles, des thiocarboxyles, des carbamyles, des thiocarbamyles, des thiocarbamides, des alcools, des thiols, des amines substitués ou non.
- Libération directe : la libération du produit obtenue directement après ladite réaction d'oxydation chimique.
- Libération indirecte : la libération du produit obtenue après une ou plusieurs réactions secondaires notamment chimique suivant ladite réaction d'oxydation chimique. La réaction secondaire peut correspondre, à titre d'exemple, à une réaction de béta élimination. Des libérations indirectes sont également celles résultant de l'exposition d'un produit obtenu directement après ladite réaction d'oxydation chimique à des irradiations ou à l'action d'enzymes plus ou moins spécifiques. Ces réactions secondaires peuvent permettre notamment le contrôle de la libération dudit produit.

Dans le cas d'une libération directe le produit libéré correspond à la formule R'₇R'₈C1=X et/ou R'₉R'₁₀C2=Y dans lesquelles R'7 à R'10, X, et Y ont les mêmes significations que précédemment.

Dans le cas d'une libération indirecte le produit libéré correspond au produit d'au moins une réaction secondaire effectuée sur au moins un composé de formule R'₇R'₈C1=X ou R'₉R'10C2=Y dans lesquelles R'₇ à R'₁₀, X, et Y ont les mêmes significations que précédemment.

Le produit libéré de manière directe ou indirecte peut être détecté, on parle alors de produit détectable correspondant à une molécule volatile détectable, à une substance active détectable ou encore à un composé spécifique détectable.

Dans le cas de la libération directe ou indirecte d'une molécule volatile, d'une substance active ou d'un composé spécifique, le composé de formule (II') permet de libérer, après l'oxydation chimique, respectivement au moins une molécule volatile, au moins une substance active ou au moins un composé spécifique.

On connaît que, par exemple, les groupes pinacols ou les groupes diols vicinaux tout comme les groupes aminoalcools ne sont pas sensibles à l'oxydation de l'air et ne sont pas volatiles. Ils peuvent donc être utilisés en tant que dérivés stables de la molécule volatile, qui sera libérée notamment de manière contrôlée par exposition desdits dérivés à un agent oxydant, tel que le periodate.

Ainsi, dans le cas où le produit libéré correspond à une molécule volatile, la méthode de l'invention peut être utilisée pour mettre en oeuvre un diffuseur d'une molécule volatile laquelle molécule volatile sera libérée progressivement à partir d'un substrat par une oxydation chimique de celui-ci.

Dans le cas où le produit correspond à une substance active, la méthode de l'invention peut être utilisée pour libérer une forme galénique d'un médicament.

La présente décrit également une composition comprenant un composé de formule (II') à partir duquel on peut libérer un produit de manière directe ou indirecte par la méthode de l'invention.

Une telle composition peut être utile, à titre d'exemple, pour la préparation d'un diffuseur d'une molécule volatile, comprenant un mélange d'au moins un composé de formule (II') et un oxydant tel que le periodate.

Selon un mode de mise en oeuvre préféré, la composition décrite dans la présente comprend une matrice solide inerte ou une forme galénique d'un médicament.

A titre d'exemple, la molécule volatile peut être un parfum ou une molécule bioactive comme une phéromone. On envisage alors respectivement un diffuseur de parfum ou un diffuseur anti-moustique.

Plus particulièrement, la molécule volatile est choisie parmi le groupe comprenant : le benzaldéhyde (amande artificielle), le butanal, le citronellal, l'anisaldehyde la menthone, le cuminaldehyde, le salicylaldéhyde (utilisé en parfumerie), la vanilline, le phenylacetaldéhyde (hyacinthe) ou l'isovaleraldéhyde (citron, menthe, eucalyptus).

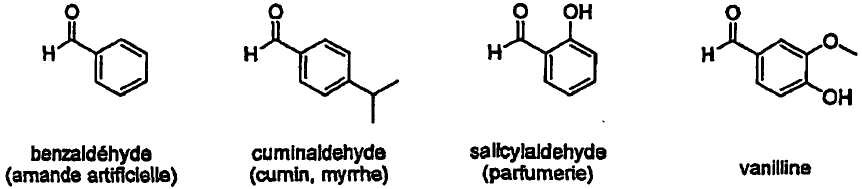

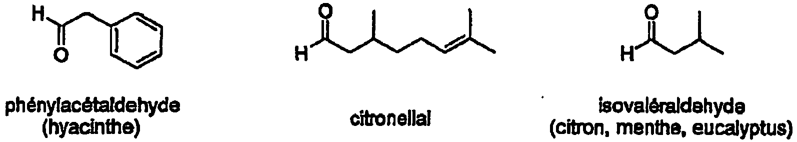

Avantageusement, la substance active telle que définie dans **la présente** est choisie parmi le groupe comprenant : la testostérone, l'oestrone ou la nicotine.

La méthode de libération du produit peut comprendre en outre une étape préliminaire de préparation du composé de formule (II') par toute technique connue de l'homme de métier.

Il s'agit par exemple de la préparation d'un dérivé stable d'une molécule volatile, ou d'une substance active ou d'un composé spécifique.

Il peut s'agir par exemple de la préparation d'un diol vicinal ou d'un aminoalcool dudit produit par toute technique connue de l'homme de métier.

Selon une mise en oeuvre particulière, **est également décrite dans la présente** une méthode de libération d'au moins un produit comprenant les étapes suivantes :
a) la préparation d'un composé de formule (II'). 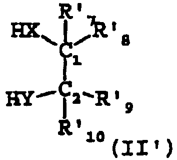 dans laquelle la liaison C1-C2 est sensible à une coupure par une réaction d'oxydation chimique, et, R'₇, R'₈, R'₉, R'₁₀, X et Y ont la même signification que précédemment,
(b) l'oxydation chimique du composé de formule (II') obtenu à l'étape (a) qui coupe la liaison C1-C2 pour obtenir ledit produit.

L'étape (a) de préparation peut comporter au moins une transformation chimique et/ou au moins une transformation enzymatique.

La préparation du composé de formule (II') peut se faire par exemple, par obtention de la forme réduite de la fonction cétone ou aldéhyde d'au moins un dudit produit de formule R'₉R'₁₀C₂=Y et/ou R'₇R'₈C₁=X

Ainsi, l'étape (a) peut être par exemple une réaction de dimérisation d'au moins une molécule volatile notamment d'un aldéhyde ou d'une cétone.

Avantageusement la réaction de dimérisation de l'étape (a) de la méthode de libération d'un produit de l'invention peut être effectuée par biosynthèse des diols vicinaux.

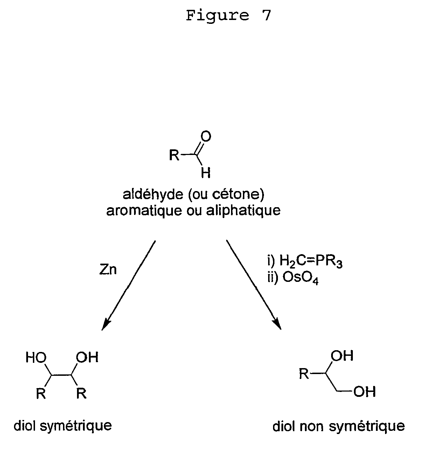

De telles réactions de dimérisation d'un produit pour obtenir un dérivé stable sont par exemple celles décrites sur la figure 7 en annexe dans laquelle :
- Une première réaction de dimérisation selon l'étape (a) est une dimérisation réductrice de l'aldéhyde 1 ou de la cétone 2 du produit libéré en présence de zinc, afin d'obtenir un groupe pinacol.
- Une deuxième réaction de dimérisation selon l'étape (a) est réalisée en deux étapes, par oléfination du carbonyle en présence d'un ylide phosphoré, suivie d'une dihydroxylation par le tétroxyde d'osmium. Cette deuxième voie conduit à un diol terminal qui, après oxydation par le periodate de sodium, libère 1 équivalent de formaldéhyde.

A titre d'exemples de synthèse d'un composé de formule (II'), dans le cas des molécules volatiles, on peut citer :

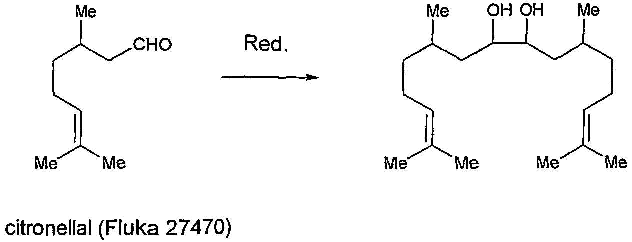

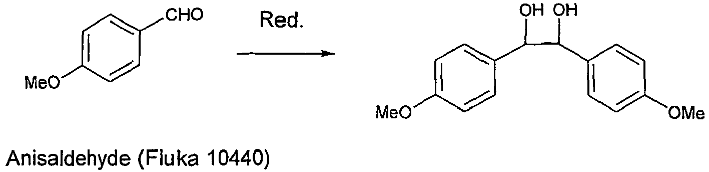

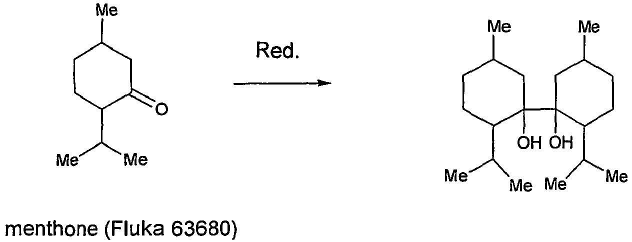

Des mises en oeuvre particulières de la méthode de libération d'une molécule volatile effectuées par la demanderesse dans le cadre de la présente invention sont détaillées dans les exemples ci-dessous dans lesquels on met en oeuvre des diols :
1 - Un diol symétrique, l'hydrobenzoïne, obtenu par réduction au borohydrure de sodium de la benzoïne, permet d'obtenir par oxydation chimique le benzaldéhyde, à l'odeur d'amande.
2 - L'oxydation du 1,2-pentanediol, est également illustrée, elle libère un aldéhyde aliphatique le butanal ayant une odeur de beurre.

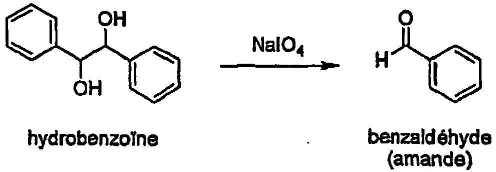

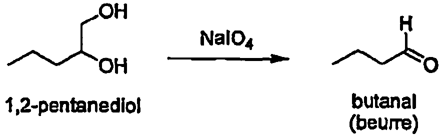

Des mises en oeuvre particulières de la méthode de libération d'une substance active sont détaillées dans les exemples ci-dessous.

Les deux premiers exemples correspondent à des libérations directes de la substance active et le dernier exemple à une libération indirecte dans laquelle la réaction d'oxydation est suivie d'une béta élimination.

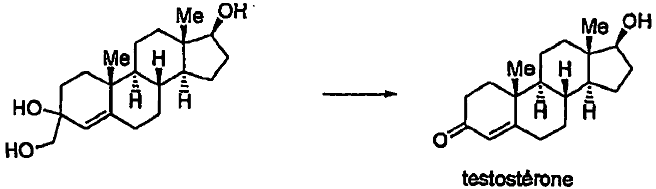

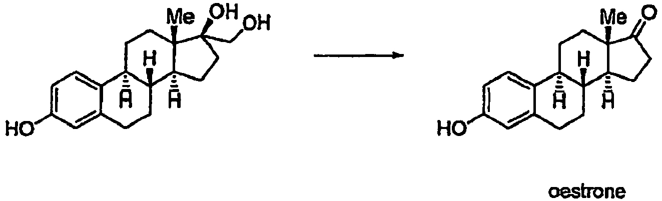

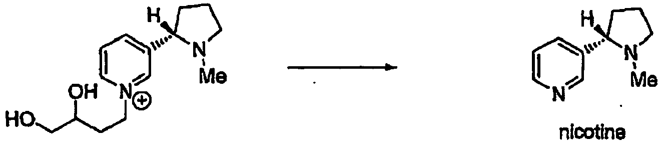

La méthode décrite dans la présente est remarquable en ce que le produit libéré est un produit détectable, elle peut alors être utilisée pour détecter une transformation chimique.

Avantageusement lorsque la libération d'un produit détectable le composé de formule (II') est un composé de formule (II) 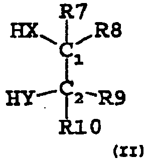 dans laquelle,
- X et Y, identiques ou différents, sont choisis parmi un atome d'oxygène, un atome de soufre, une amine de formule -NR₁₁R₁₂, R₁₁ est choisi parmi: un atome d'hydrogène, un groupe alkyle, un groupe aryle, substitués ou non, et R₁₂ n'est pas un atome d'hydrogène, - R₇ à R₁₀, identiques ou différents sont choisis parmi un atome d'hydrogène, un groupe alkyle substitué ou non, ou un groupe fonctionnel substitué ou non, et au moins un des groupes R₇ à R₁₀ est un groupement Di, qui est insensible à la réaction d'oxydation chimique de l'étape (b) et qui présente des propriétés détectables, directement ou indirectement, seul ou en combinaison, après la coupure de la liaison C₁-C₂.

Ces propriétés sont caractéristiques du produit détectable.

A titre d'exemple ces propriétés peuvent être biophysiques, biologiques ou chimiques, telles que notamment une variation de type spectrale ou une variation de solubilité.

Le composé de formule (II) correspond à une mise en oeuvre particulière du composé de formule (II'), ainsi les considérations faites pour le composé de formule (II') sont valables pour le composé de formule (II).

Le produit détectable peut alors être libéré après avoir effectué une première étape de transformation chimique d'un substrat pour obtenir un composé de formule (II) puis une deuxième étape d'oxydation chimique dudit composé de formule (II).

La présente invention concerne donc tout particulièrement une méthode de détection d'une transformation chimique d'un substrat telle que ladite transformation chimique de ce substrat libère un produit. Ledit produit libéré étant alors un produit détectable.

L'invention concerne également l'utilisation de substrats susceptibles d'être utilisés dans cette méthode. L'invention concerne encore l'utilisation de cette méthode dans un procédé de détection et éventuellement de quantification d'une transformation chimique dans un échantillon.

De nombreux substrats libèrent après réaction un produit donnant un signal facilement identifiable. On peut citer les exemples bien connus des glycosides, des esters et des phosphates de phénols dont l'hydrolyse libère directement le phénol, qui est détecté en général par sa couleur (substrat chromogène) ou sa fluorescence (substrat fluorogène) (D. C. Demirjian, P. C. Shah, F. Moris-Varas, Top. Curr. Chem. 1999, 200, 1 ; M. T. Reetz, K.-E. Jaeger, Top. Curr. Chem. 1999, 200, 31).

On peut citer également le 2-méthoxy-1-naphtalèneméthanol, dont l'oxydation donne le 2-méthoxy-1-naphtaldéhyde fluorescent (B. L. Vallee, brevet US 5,162,203 ; B. List, C. F. Barbas, R. A. Lerner, Proc. Natl. Acad. Sci. USA 1998, 95, 15351 for utilisation in aldolases).

Ces types de substrat ont un désavantage majeur : le phénol chromogène ou fluorogène est un groupe fortement activé, ce qui rend ces substrats instables et susceptibles de réactions non-spécifiques. Dans le cas du 2-méthoxy-1-naphtalèneméthanol, le problème est similaire puisque la position benzylique est sensible à l'oxydation non-spécifique.

Une deuxième classe de substrat conduit à la révélation d'un produit obtenu après réaction secondaire enzymatique et/ou spontanée. Il existe quelques exemples, comme des substrats de pénicillinase (Brevet US 5,583,217), des substrats d'alcool déhydrogenase (ADH) (G. Klein, J.-L. Reymond, Bioorg. Med. Chem. Lett. 1998, 8, 1113) et des substrats d'aldolase (N. Jourdain, R. Pérez Carlón, J.-L. Reymond, Tetrahedron Lett. 1998, 39, 9415).

On peut également noter l'existence d'un substrat pour mesurer le NADH (C. A. Roeschlaub, N. L. Maidwell, M. R. Rezai, P. G. Sammes, Chem. Commun. 1999, 1637).

Enfin, on peut citer des substrats dont le produit de la réaction secondaire détecté est obtenu par l'action de la beta-galactosidase (K.L. Matta, C.F. Piskorz, J.J. Barlow, Carbohydr.Res. 1981, 90, C1-C3) ou par l'ADH (G.Klein, J.-L. Reymond, Helv.Chim.Acta 1999, 82, 400).

Cette deuxième classe de substrat est plus stable. Cependant, cette deuxième classe de substrats est limitée à des utilisations particulières, car le fait d'utiliser une enzyme pour la réaction secondaire représente un inconvénient en terme de coût et de flexibilité.

Les travaux de recherche effectués dans le cadre de la présente invention ont notamment consisté à développer une méthode de détection d'une transformation chimique. Ainsi, la présente invention se propose de résoudre les inconvénients rapportés ci-dessus en utilisant une méthode de mesure d'une transformation chimique fiable, sensible et reproductible, mettant en oeuvre un substrat stable dans le milieu dans lequel se produit la réaction chimique, et tel que les transformations successives libèrent un produit détectable.

La détection de cette transformation est obtenue en utilisant la méthode de libération d'un produit détectable soit en effectuant une transformation chimique d'un substrat conduisant au composé de formule (II), puis en oxydant chimiquement le composé de formule (II) pour libérer le produit pouvant être détecté. Dans ce cas la libération du produit détectable comprend les étapes suivantes :
a) la transformation chimique d'un substrat de formule (I) dans laquelle la liaison C1-C2 est insensible à une coupure par une réaction d'oxydation chimique **effectué au moyen d'un agent chimique oxydant**: 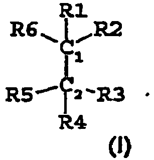 en un composé de formule (II) dans laquelle la liaison C₁-C₂ est sensible à une coupure par une réaction d'oxydation chimique : 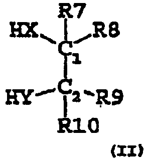 et,
(b) l'oxydation chimique du composé de formule (II) obtenu à l'étape (a) qui coupe la liaison C1-C2 pour obtenir directement ou indirectement un produit détectable,
et en ce que dans les composés de formules (I) et (II) :
- au moins un des groupes R₁ à R₆ est un groupement Di tel que défini précédemment,
- les groupes R₁ à R₆, identiques ou différents, sont choisis parmi: un atome d'hydrogène, un groupe alkyle substitué ou non, un groupe fonctionnel substitué ou non et dans le cas d'un groupe fonctionnel de formules -OR₁₂, -SR₁₂ , -NR₁₁R₁₂, R₁₁ est choisi parmi: un atome d'hydrogène, un groupe alkyle, un groupe aryle, substitués ou non, et R₁₂ n'est pas un atome d'hydrogène,
- le ou les groupes R₁ à R₆ formant après l'étape (a) les groupes de formules -XH et -YH sont insensibles à l'oxydation de l'étape (b), ou peuvent être sensibles à cette oxydation si le composé de formule (I) correspondant ne conduit pas lors d'une oxydation du type de celle de l'étape (b) au composé de formule (II),
- X et Y, identiques ou différents, sont choisis parmi un atome d'oxygène, un atome de soufre, une amine de formule -NR₁₁R₁₂,
- R₇ à R₁₀, identiques ou différents, sont, soit identiques à quatre au plus des groupes R₁ à R₆, soit, du fait de la transformation d'un ou plusieurs des groupes R₁ à R₆, lors de la réaction de l'étape (a), choisis parmi un atome d'hydrogène, un groupe alkyle substitué ou non, ou un groupe fonctionnel substitué ou non, et au moins un des groupes R₇ à R₁₀ est un groupement Di tel que défini précédemment.

La méthode de l'invention comprend évidemment après l'étape (b) une étape de détection directe ou indirecte du produit détectable, pour effectuer par exemple la détection de la transformation chimique de l'étape (a).

La méthode de l'invention accepte plusieurs significations préférées des groupes R1 à R6, parmi lesquelles on envisage plus particulièrement les cas suivants:
- R₁ à R₆ sont choisis de façon à ce que la liaison C₁-C₂ fasse partie d'au moins un cycle,
- au moins une paire des groupes R₁ à R₆ forment ensemble un atome d'oxygène, un atome de soufre, ou un groupe de formule -NR11R12,
- au plus une paire de groupes R₁ à R₆ dont l'un est choisi parmi R₁, R₂ et R₆ et l'autre choisi parmi R₃, R₄ et R₅ forment une double liaison entre les carbones C₁ et C₂,
- au plus deux paires de groupes R₁ à R₆ dont l'un est choisi parmi R₁, R₂ et R₆ et l'autre choisi parmi R₃, R₄ et R₅ forment une triple liaison.

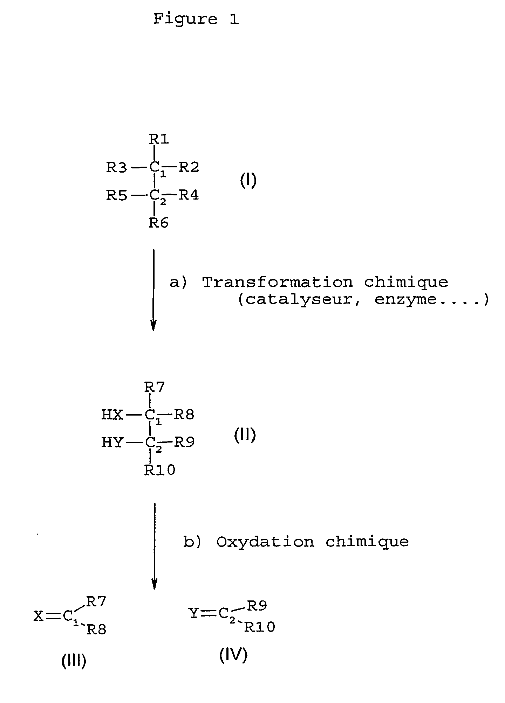

Un exemple de schéma de détection d'une transformation chimique d'un substrat selon l'invention est représenté à la figure 1 en annexe. Les composés des formules (III) et (IV) étant directement ou indirectement détectables.

Une forme avantageuse de mise en oeuvre de la méthode de libération d'un produit détectable comprend les étapes suivantes :
(a) la transformation d'un substrat de formule (I') dans laquelle la liaison C1-C2 est insensible à une coupure par une réaction d'oxydation chimique : 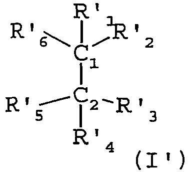 en un composé de formule (II') dans laquelle la liaison C₁-C₂ est sensible à une coupure par une réaction d'oxydation chimique: 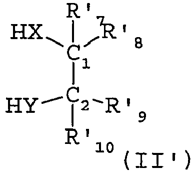 et,
(b) l'oxydation chimique du composé de formule (II') obtenu à l'étape (a) en présence d'un composé de formule II, qui coupe la liaison C1-C2 pour obtenir directement ou indirectement au moins un produit détectable,
où, dans le composé de formule (I'):
- les groupes R'₁ à R'₆, identiques ou différents, sont choisis parmi: un atome d'hydrogène, un groupe alkyle substitué ou non, un groupe fonctionnel substitué ou non et dans le cas d'un groupe fonctionnel de formules -OR₁₂, -SR₁₂ , -NR₁₁R₁₂, R₁₁ est choisi parmi: un atome d'hydrogène, un groupe alkyle, un groupe aryle, substitués ou non, et R₁₂ n'est pas un atome d'hydrogène,
- le ou les groupes R'₁ à R'₆ formant après l'étape (a) les groupes de formules -XH et -YH sont insensibles à l'oxydation de l'étape (b), ou peuvent être sensibles à cette oxydation si le composé de formule (I') correspondant ne conduit pas lors d'une oxydation du type de celle de l'étape (b) au composé de formule (II'),
dans le composé de formule (II'):
- X et Y, identiques ou différents, sont choisis parmi un atome d'oxygène, un atome de soufre, une amine de formule -NR₁₁R₁₂, R₁₁ est choisi parmi: un atome d'hydrogène, un groupe alkyle, un groupe aryle, substitués ou non, et R₁₂ n'est pas un atome d'hydrogène.
- R'₇ à R'₁₀, identiques ou différents, sont, soit identiques à quatre au plus des groupes R'₁ à R'₆, soit, du fait de la transformation d'un ou plusieurs des groupes R₁ à R₆, lors de la réaction de l'étape (a), choisis parmi un atome d'hydrogène, un groupe alkyle substitué ou non, ou un groupe fonctionnel substitué ou non et,
dans le composé de formule (II) :
- R₇ à R₁₀, identiques ou différents, correspondent à un atome d'hydrogène, un groupe alkyle substitué ou non, un groupe fonctionnel substitué ou non et au moins un des groupes R₇ à R₁₀ est un groupement Di tel que défini précédemment.
- X et Y, identiques ou différents, sont choisis parmi un atome d'oxygène, un atome de soufre, une amine de formule -NR11R12, R11 est choisi parmi: un atome d'hydrogène, un groupe alkyle, un groupe aryle, substitués ou non, et R12 n'est pas un atome d'hydrogène.

Cette forme de mise en oeuvre avantageuse met en compétition le composé de formule (II'), issu de la transformation chimique du composé de formule (I'), avec le composé de formule (II) lors de l'étape (b) d'oxydation. La vitesse d'oxydation du composé de formule (II) va donc diminuer proportionnellement à la quantité de composé de formule II' formé. Le signal associé à la transformation du composé de formule (II), détectable grâce à la présence d'un groupement Di précurseur d'un produit détectable va donc aussi diminuer proportionnellement à la quantité de composé de formule (II') produit.

De cette manière, on peut détecter par l'intermédiaire du composé de formule (II) la réaction chimique transformant le composé de formule (I') en composé de formule (II').

Dans cette forme de mise en oeuvre avantageuse, le substrat de formule (I') peut correspondre au substrat spécifique non-modifié de la transformation chimique comme par exemple le substrat spécifique non-modifié d'une enzyme.

La méthode de l'invention accepte également une modification d'un des groupes R₇ à R₁₀ en dehors de Di, par l'oxydant chimique de l'étape (b), à condition que cela ne perturbe pas la coupure de la liaison C1-C2 lors de l'étape (b) ni lors de la détection ultérieure.

Dans une forme particulière de mise en oeuvre du procédé de l'invention où les étapes (a) et (b) sont simultanées, le ou les groupes R₁ à R₆ sont insensibles à l'oxydation de l'étape (b).

Dans ces conditions, la réaction de transformation chimique et d'oxydation chimique ont lieu dans les mêmes conditions expérimentales. Lorsque les étapes (a) et (b) sont effectuées simultanément, le profil du signal obtenu peut renseigner sur le déroulement cinétique de la transformation, si celle-ci est limitante dans le processus, c'est-à-dire plus lente que l'oxydation.

Dans une autre forme de mise en oeuvre, les étapes (a) et (b) sont non simultanées. Les groupes R₁ à R₆ sont alors sensibles ou non à l'oxydation de l'étape (b). La réaction de transformation (a) peut avoir lieu dans des conditions expérimentales semblables ou non à la réaction d'oxydation (b). Lorsque les étapes (a) et (b) sont non simultanées on obtiendra une vitesse de réaction proportionelle à la quantité de produit accumulé.

La méthode de l'invention peut être mise en oeuvre avec un substrat comprenant plusieurs groupes chimiques susceptibles d'être transformés à l'étape (a) et plusieurs groupes chimiques précurseurs de produits détectables.

Selon la transformation chimique étudiée et/ou la structure du substrat utilisée, la méthode de l'invention pourra être réalisée dans un milieu réactionnel adéquat choisi parmi un milieu aqueux, organique, biphasique ou solide.

Dans ce cas, on entend par :
- transformation chimique ou réaction chimique, toute transformation d'un substrat qui peut être spontanée ou non et qui peut nécessiter des conditions expérimentales particulières comme la chaleur, les UV, etc. Dans le cas d'une transformation chimique non spontanée, la réaction peut nécessiter un réactif chimique ou un catalyseur comme une enzyme. Cette transformation chimique de l'étape (a) peut être réalisée au moyen de plusieurs réactions séquentielles, simultanées ou non.

Les groupes R₁ à R₁₀ quelle que soit leur signification peuvent être constitués ou comprendre un isotope comme par exemple le deutérium. En outre, avantageusement, les groupes R1 à R10 sont stables dans un milieu réactionnel, notamment aqueux, organique, biphasique, solide, etc.

Avantageusement, le substrat mis en oeuvre dans le procédé de l'invention comprend un ou plusieurs centres chiraux. Le substrat mis en oeuvre dans le procédé de l'invention peut donc être constitué d'un mélange d'énantiomères, ou de diastéréoisomères, ou d'énantiomères purs.

Des substrats préférés répondant à la formule I sont décrits ci-après.

Une première classe de substrat (I) répond aux formules (V) à (IX) suivantes : 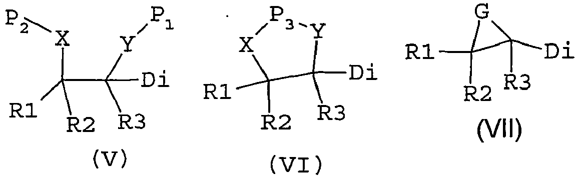 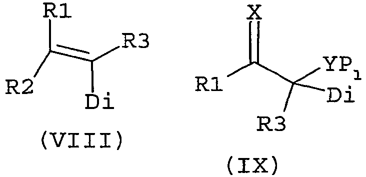 dans lesquelles :
- Di est un groupement tel que défini précédemment
- R₁ à R₃ et X et Y ont les mêmes significations que précédemment,
- Au plus un des groupes P1 et P2 est un atome d'hydrogène. P₁ P₂ , identiques ou différents sont choisis parmi, un groupe acyle substitué par un groupe aryle ou alkyl ou peptidyle, un groupe phosphate, un groupe ester phosphate, un groupe phosphonate, un groupe carbamyle substitué par un groupe aryle ou acyle ou peptidyle, un groupe glycosyle et un groupe sulfate,
- P₃ est choisi parmi un groupe carbonyle, un groupe -PO₂R₁₁ ou un groupe R₁₁PO-, où R₁₁ présente la même signification que précédemment, un groupe -SO₂, un groupe - CHOR₁₃ où R₁₃ représente un groupe aryle, alkyle ou glycosyle, un groupe SiR₁₄R₁₅ où R₁₄ et R₁₅, identiques ou différents, représentent un groupe aryle, alkyle, aryloxy ou alcoxy et un groupe AsO₂H-.
- G est choisi parmi un atome d'oxygène, un atome de soufre, un groupe amine de formule NR11R12 où R₁₁ a la même signification que précédemment.

Le produit du substrat obtenu après la première étape de la méthode de l'invention à partir des substrats de formules (V), (VI), (VII) (VIII) et (IX) répond à la formule (II) ou avantageusement à la formule (X) suivante : 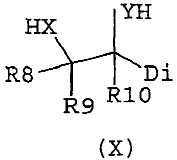 dans laquelle, Di, X, Y, R₈, R₉, et R₁₀ ont la même signification que précédemment.

Dans un cas particulier, le produit du substrat obtenu après la première étape (a) de transformation chimique de l'invention à partir des substrats de formules (I) peut également répondre à la formule (XV) suivante : 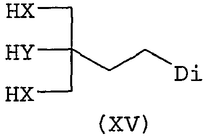 dans laquelle X, Y et Di ont la même signification que précédemment , la chaîne portant Di peut être en position 1, 2 ou 3 du dérivé glycérolé.

Tout à fait préférentiellement, les groupes X et Y du composé de formule (XV) sont des groupes hydroxyles.

La détection de la transformation chimique selon l'invention est mise en oeuvre soit par une révélation directe, soit par une révélation indirecte du composé obtenu après l'oxydation chimique du composé de formules (II) ou (X) effectué au moyen d'un agent chimique oxydant selon l'étape (b).

Le tétrapropyle ammonium periodate, soluble en phase organique, est avantageusement utilisé lorsque la méthode de l'invention est réalisée en milieu organique.

Selon un mode de révélation préférée ci-après nommée révélation directe, selon laquelle le composé répondant à la formule (XI) obtenu après la réaction d'oxydation chimique effectuée sur le dérivé répondant aux formules (II) ou (X) après l'étape (b), est directement détectable, selon le schéma ci-dessous.

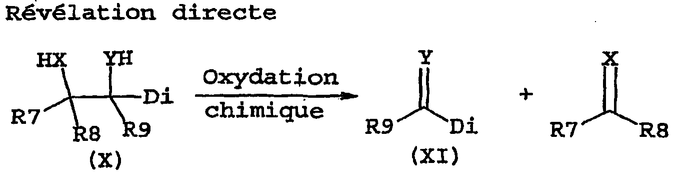

Un mode de réalisation de l'invention avec une révélation directe met en oeuvre la détection directe d'une propriété du composé de formule (XI), non présente sur les composés de formules (I) ou (II) ou (X).

Dans un mode de révélation directe ou de libération directe d'un produit détectable :
- Di dans le composé de formule (X) est un précurseur d'un produit détectable, et
- Di dans le composé de formule (XI) est directement détectable.

A titre d'exemples non limitatifs on peut citer parmi ces propriétés du composé (XI), une propriété physique, telle que la solubilité, une propriété physicochimique, telle qu'une propriété spectrale, ou une propriété biologique, telle que l'activation d'une enzyme, une odeur, ou l'action d'une phéromone.

La molécule répondant à la formule (XI) peut correspondre à des cétones aromatiques, par exemple une cétone béta-aromatique que l'on détecte par une variation spectrale, un aldéhyde comme le benzaldéhyde ou le citronellal que l'on détecte par l'odeur ou à une phéromone que l'on détecte par l'attraction d'insectes. (Suzuki et al., (1980). Agric.Biol.Chem. 44, 2519 et Millar et al. (1996). Bioorg.Med.Chem. 3, 331-340).

Dans un deuxième mode de révélation préférée, ci-après nommé de révélation indirecte, le composé (XII), obtenu après l'étape (b) d'oxydation chimique du composé de formules (II) ou (X), subit une réaction de béta-elimination conduisant au produit détectable ZH, selon le schéma ci-dessous.

### Révélation indirecte

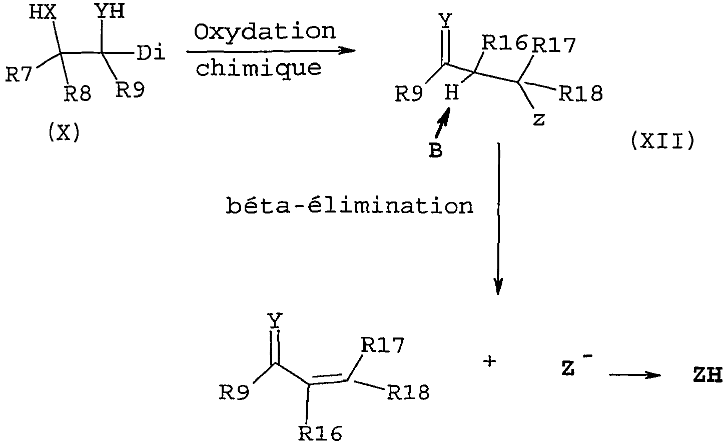

le groupe Di, répondant à la formule (XIII) suivante: 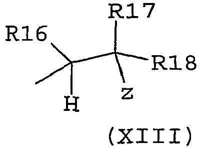 dans laquelle :
- R₁₆, R₁₇ et R₁₈, identiques ou différents, représentent un atome d'hydrogène, un groupe alkyle substitué ou non ou un groupe fonctionnel substitué ou non, et
- Z est un précurseur du produit détectable ZH.

Dans ce mode de révélation indirecte ou de libération indirecte d'un produit détectable :
- Di dans les composés de formules (X) et (XII) est le précurseur dudit produit détectable qui sera libéré après une réaction secondaire de béta-élimination.

Dans ce mode de réalisation de l'invention avec une révélation indirecte, on réalise la détection d'une propriété du produit ZH obtenu par une réaction de béta-élimination effectuée sur le composé de formule (XII) obtenu après l'étape (b) d'oxydation chimique. Cette réaction de béta-élimination, avantageusement spontanée, est réalisée préférentiellement en présence d'une base appelée B qui peut correspondre à l'albumine de sérum de boeuf (BSA).

Parmi les propriétés du composé ZH, on peut citer à titre d'exemples non limitatifs une propriété physique telle que la solubilité, une propriété physicochimique telle qu'une propriété spectrale ou une propriété biologique telle que l'induction de croissance de bactéries.

Le composé ZH est choisi parmi un alcool aromatique, un alcool hétéroaromatique, une amine hétéroaromatique, un atome d'halogène, ou un ester phosphorique. A titre d'exemples non limitatifs peuvent être cités, la fluorescéine, la phenolphtaléine, le rouge de phénol, le p-nitrophénol l'o-nitrophénol, le 2,4-dinitrophénol, l'acide 6-hydroxynaphtoïque, l'acide 8-hydroxy-pyrène 1,3,6-trisulfonique, la tyrosine, la luciférine, l'indolyle, le 5-bromo-4-chloro-indolyle, le quinolinium, le nitro-anilinium, ou la pyridoxamine.

Dans un troisième mode de révélation préférée, le composé obtenu après l'étape (a) de transformation chimique du substrat répond à la formule XIV suivante : 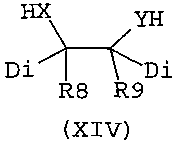 dans laquelle :
- Di, R8, R9, X et Y ont les mêmes significations que précédemment et,
- les deux groupes Di identiques ou différents, l'un attaché au carbone C₁, le deuxième attaché au carbone C₂ interagissent entre eux.

Dans ce cas, la coupure de la liaison C₁-C₂ sur ce composé (XIV) provoque une variation spectrale détectable.

Un exemple non limitatif d'interaction entre les deux groupes Di est un transfert d'énergie de type FRET.

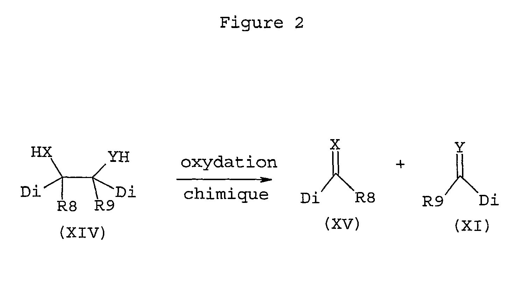

Un schéma représentant cette révélation mettant en oeuvre un transfert d'énergie de type FRET est représenté figure 2 en annexe.

Des exemples de substrats permettant la mise en oeuvre de l'invention avec une révélation directe sont des composés de formule (V) dans lesquelles les groupes X, Y, R₁, R₂, R₃ et Di sont choisis parmi ceux décrits dans le tableau suivant.

Pour tous les composés décrits dans les tableaux suivants, illustrant des exemples particuliers de substrats selon l'invention, la stéréochimie est toujours réalisée dans toutes les variantes possibles.

**Tableau I**

| N° | X | Y | R₁ | R₂ | R₃ | Di |
|---|---|---|---|---|---|---|
| 6 | NH | O | H | H | H | 6'-methoxy-2'-naphtyl |
| 7 | NH | O | COOH | H | H | 6'-methoxy-2'-naphtyl |
| 8 | NH | O | CONH-peptide | H | H | 6'-methoxy-2'-naphtyl |
| 17 | O | O | H | H | H | 6'-methoxy-2'-naphtyl |

Le groupe 6'-methoxy-2'-naphtyl correspond à la formule :

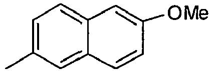

Des exemples de substrats permettant la mise en oeuvre de l'invention avec une détection indirecte sont des composés de formule (V) dans lesquelles les groupes X, Y; R₁₁, R₁₂, R₁₃, ainsi que les groupes R₁, R₂, R₃ et ZH sont choisis parmi ceux décrits dans le tableau II suivant.

**Tableau II**

| | | | | | | Di | | | |
|---|---|---|---|---|---|---|---|---|---|
| N° | X | Y | R₁ | R₂ | R₃ | R₁₆ | R₁₇ | R₁₈ | Z |
| 1 | NH | O | H | H | H | H | H | H | Coum*/NP** |
| 2 | NH | O | CH₂ | H | H | H | CH₂ | H | Coum*/NP** |
| 3 | NH | O | COOH | H | H | H | H | H | Coum*/NP** |
| 4 | NH | O | CONH-peptide | H | H | H | H | H | Coum*/NP** |
| 5 | NH | O | H | H | Me | H | H | H | Coum*/NP** |
| 9 | O | O | H | H | H | H | H | H | Coum*/NP** |
| 10 | O | O | Me | Me | H | H | H | H | Coum*/NP** |
| 11 | O | O | H | CH₂ | H | H | CH₂ | H | Coum*/NP** |
| 12 | O | O | (MeO)CHO | H | H | O-CH(OMe) | H | H | Coum*/NP** |
| 13 | O | O | H | H | Me | H | H | H | Coum*/NP** |
| 14 | O | O | Et | H | H | H | H | H | Coum*/NP** |
| 15 | O | O | (MeO)CHO | H | H | O-CH(OMe) | H | H | Coum*/NP** |
| 16 | O | O | (Ade)CHO | H | H | O-CH(Ade) | H | H | Coum*/NP** |
| 18 | NH | NH | H | H | H | H | H | H | Coum*/NP** |

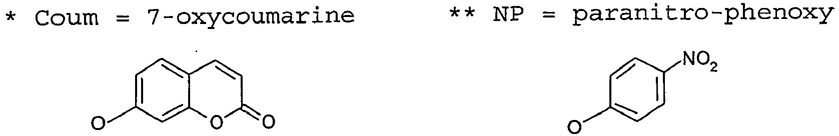

Les structures correspondantes aux substrats du tableau II sont représentées ci-après:
- Aminoalcools 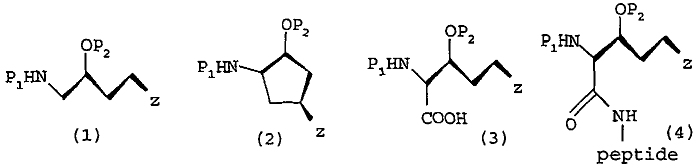 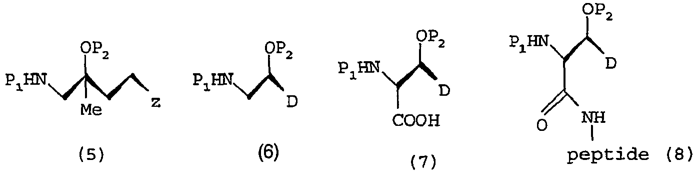
- Diamines

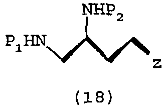

Les diamines peuvent être mono ou disubstituées, les groupes P1 et P2 ne pouvant pas correspondre tous les deux à H.
P1 et/ou P2 peuvent correspondre à: 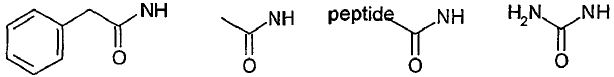 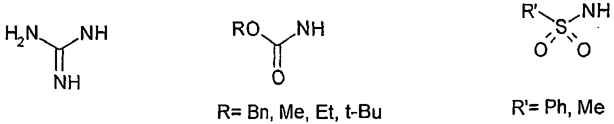
- Diols: 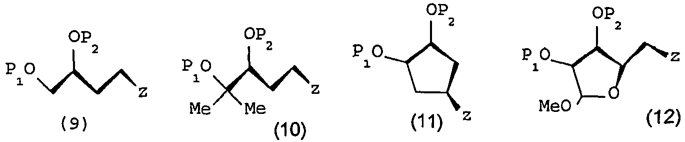 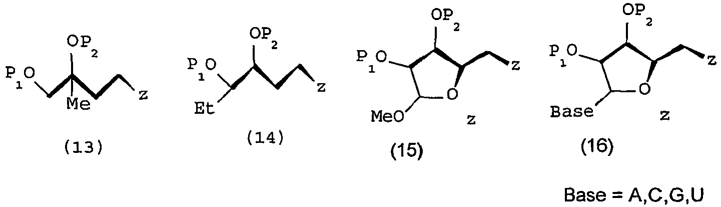
Les diols peuvent être mono ou disubstitués.

Dans le cas particulier d'une disubstitution, les deux radicaux hydroxyle peuvent être substitués identiquement comme suit:

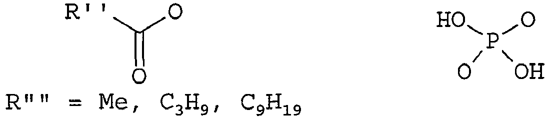

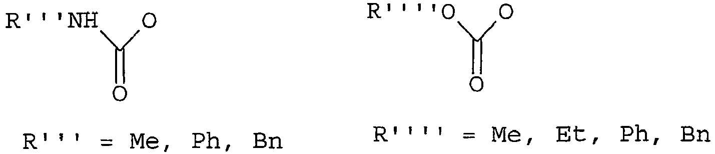

Dans le cas d'une monosubstitution, à titre d'exemple particulier, les radicaux hydroxyle peuvent être monofonctionalisés comme ci-dessus, et en plus comme suit:

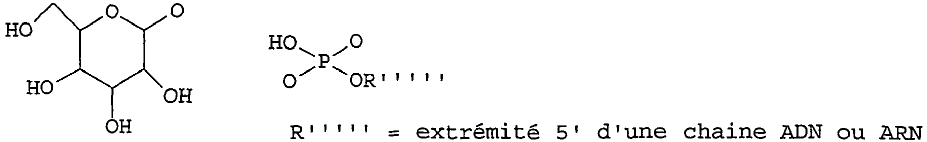

A titre d'exemple particulier de mise en oeuvre de l'invention, la révélation de l'étape (b) est réalisée de manière séquentielle en utilisant des substrats au moins disubstitués comme le substrat disubstitué de la formule XVII suivante, 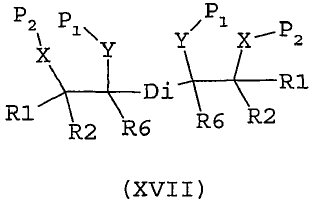 dans laquelle : Di, R1, R₂, R₆, P₁, P₂, X et Y ont les mêmes significations que précédemment.

Un mode préféré de réalisation de la méthode de l'invention mettant en oeuvre un substrat répondant à la formule (V) ci-dessus, comprend la transformation de dérivés mono ou diesters des diols (1 a/b) par une lipase ou une estérase pour conduire au composé (2) conformément au schéma ci-dessous.

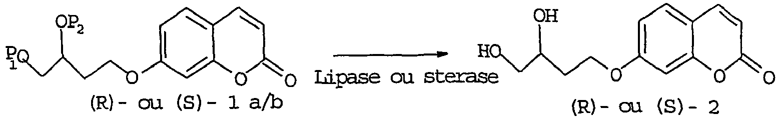

Un autre mode préféré de réalisation de la méthode de l'invention mettant en oeuvre un substrat appartenant à la deuxième classe ci-dessus, comprend la transformation de dérivés mono- ou diesters des diols (3 a/b) par une lipase ou une estérase pour conduire au composé (4) conformément au schéma ci-dessous.

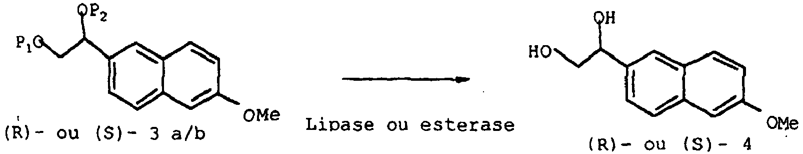

A titre d'exemple de substrats de formule (VI), on peut citer plus particulièrement ceux correspondant aux formules ci-dessous ;

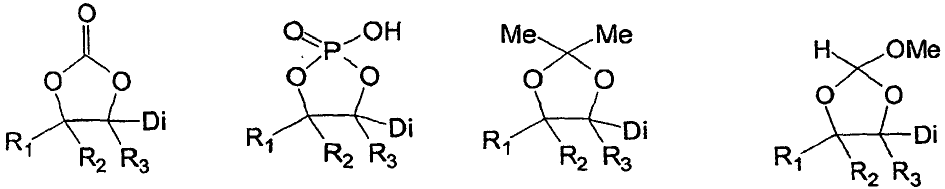

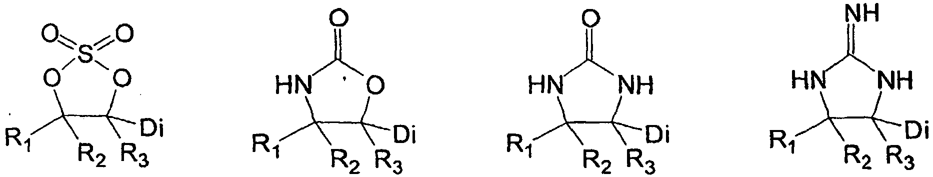

A titre d'exemple de substrats de formule (VII) mis en oeuvre dans la méthode de l'invention avec une révélation directe on peut citer plus particulièrement ceux décrits dans le tableau III suivant:

**Tableau III**

| | | | | Di | | | |
|---|---|---|---|---|---|---|---|
| N° | R₁ | R₂ | R₃ | R₁₆ | R₁₇ | R₁₈ | Z |
| 19 | H | H | H | H | H | H | coum/NP |
| 20 | H | H | Me | H | H | H | coum/NP |
| 21 | Me | Me | H | H | H | H | coum/NP |
| 22 | Et | H | H | H | H | H | coum/NP |
| 23 | CH₂- | H | H | H | CH₂- | H | coum/NP |

Coum et NP ont le mêmes significations que précédemment.

A titre d'exemple de substrats de formule (VII) mis en oeuvre dans la méthode de l'invention avec une révélation indirecte on peut citer plus particulièrement ceux décrits dans le tableau IV suivant:

**Tableau IV**

| N° | R₁ | R₂ | R₃ | Di |
|---|---|---|---|---|
| 24 | H | H | H | napht |

Les structures correspondantes aux substrats des tableaux III et IV sont représentées ci-après&

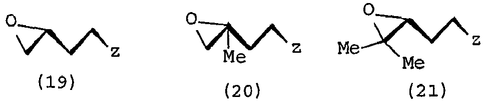

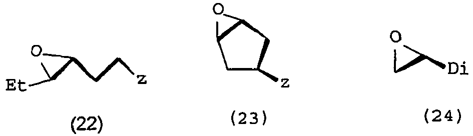

Un mode préféré de réalisation de la méthode de l'invention mettant en oeuvre un substrat répondant à la formule (VII) ci-dessus, comprend la transformation de l'époxyde (7) par une époxyde estérase pour conduire au composé (2) conformément au schéma ci-dessous.

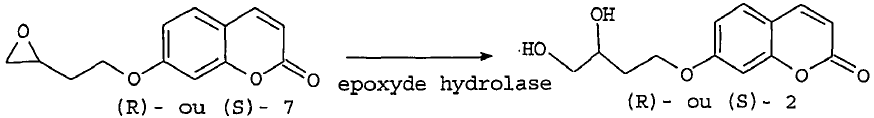

Un autre mode préféré de réalisation de la méthode de l'invention mettant en oeuvre un substrat répondant à la formule (VII) ci-dessus, comprend la transformation de l'époxyde (8) par un époxyde hydrolase pour conduire au composé (4) conformément au schéma ci-dessous.

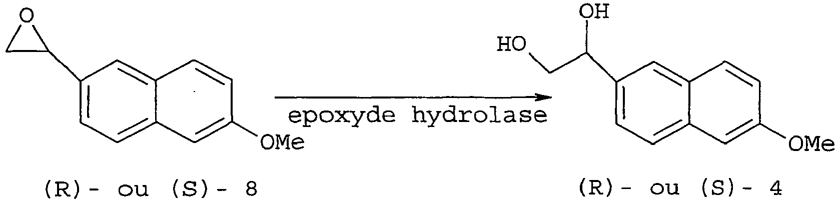

A titre d'exemple de substrats de formule (VIII) mis en oeuvre dans la méthode de l'invention avec une révélation indirecte on peut citer plus particulièrement ceux décrits dans le tableau V suivant, où les groupes coum et NP ont le mêmes significations que précédemment.

**Tableau V**

| | | | | Di | | | |
|---|---|---|---|---|---|---|---|
| N° | R₁ | R₂ | R₃ | R₁₆ | R₁₇ | R₁₈ | Z |
| 25 | H | H | H | H | H | H | coum/NP |
| 26 | H | H | Me | H | H | H | coum/NP |
| 27 | Me | Me | H | H | H | H | coum/NP |
| 28 | Et | H | H | H | H | H | coum/NP |
| 29 | CH₂- | H | H | H | CH₂- | H | coum/NP |

A titre d'exemple de substrats de formule (VIII) mis en oeuvre dans la méthode de l'invention avec une révélation directe on peut citer plus particulièrement ceux décrits dans le tableau VI suivant:

**Tableau VI**

| N° | R₁ | R₂ | R₃ | Di |
|---|---|---|---|---|
| 30 | H | H | H | napht |

Les structures correspondantes aux substrats des tableaux V et VI sont représentées ci-après

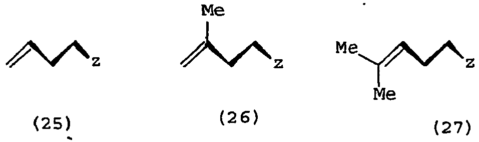

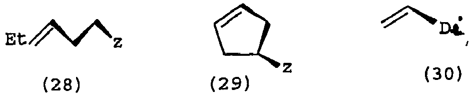

Un mode préféré de réalisation de l'étape (a) de la méthode de l'invention mettant en oeuvre un substrat selon la formule (VIII) ci-dessus, comprend la transformation de l'oléfine (9) par une dihydroxylase ou un catalyseur chimique tel que un alcaloïde ou un aminoalccol en présence de OsO4 pour conduire au composé (2) conformément au schéma ci-dessous :

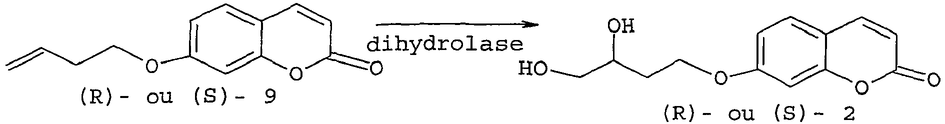

Un autre mode préféré de réalisation de l'étape (a) de la méthode de l'invention mettant en oeuvre un substrat selon la formule (VIII) ci-dessus, comprend la transformation de l'oléfine (10) par une dihydroxylase pour conduire au composé (4) conformément au schéma ci-dessous.

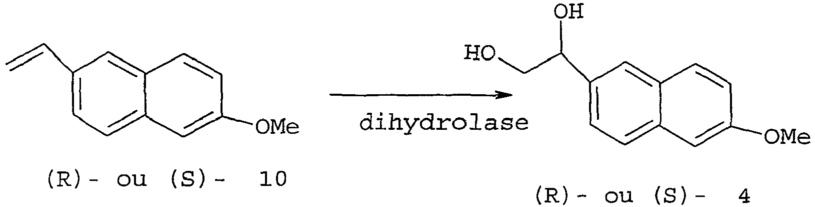

A titre d'exemple de réaction d'oxydation, selon l'étape (b), des produits issus de l'étape (a), on peut citer les deux réactions suivantes:

Avec une méthode de révélation directe 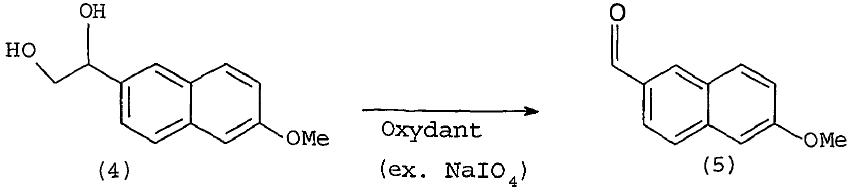 Avec une méthode de révélation indirecte

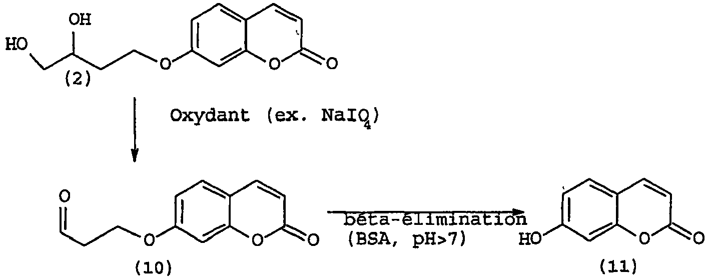

**La présente décrit également** aussi les substrats répondant aux formules (V), (VI), (VII), (VIII) ou (IX).

Ces substrats présentent un intérêt particulier et notamment, il sont susceptibles d'être mis en oeuvre dans la méthode décrite précédemment.

Avantageusement ces substrats sont stables dans le milieu réactionnel, notamment sur des gammes de pH et de températures très larges, mais également dans des solvants très divers.

A titre d'exemple, des substrats susceptibles d'être mis en oeuvre dans la méthode de l'invention sont représentés dans le tableau XI en annexe.

L'invention **décrit également** une composition comprenant au moins un composé de formule (II')

Ce composé de formule (II') peut correspondre à une forme stabilisée d'une molécule volatile, d'une substance active ou d'un composé spécifique.

Avantageusement la composition selon l'invention comprend un agent oxydant.

La méthode de l'invention est remarquable en ce qu'elle permet de détecter une transformation chimique éventuellement présente dans un échantillon en choisissant un substrat le plus adapté à la transformation que l'on souhaite analyser.

Avantageusement, ces substrats présentent des degrés de spécificité divers. Ces degrés de spécificité sont apportés par la structure d'un ou des groupes R₁ à R₆ de la formule (I), ou R₁ à R₃ ou G ou P₁ à P₃ des formules (V), (VI), (VII), (VIII) ou (IX).

A titre d'exemple, dans le cas de la détection d'une transformation chimique effectuée par une lipase, le ou les groupes R₁ à R₆ sont choisis pour que les substrats répondant à la formule (I) soient proches de la structure du substrat spécifique. En particulier ils peuvent correspondre à une chaîne d'acide gras. L'homme du métier saura, en fonction du type de transformation chimique à détecter, adapter le choix de ou des groupes R₁ à R₆. Dans le cas de mise en oeuvre de la méthode de l'invention, où deux composés de formule (II) sont mis en compétition au niveau de l'étape (2), le substrat de formule (I) peut correspondre au substrat spécifique non-modifié de l'enzyme, ce qui représente un avantage dans le cas de l'utilisation de la méthode de l'invention pour l'identification de nouveaux catalyseurs ou de l'activité nouvelle de catalyseurs connus susceptibles de transformer un substrat de formule (I) ou (I') respectivement en un composé de formule (II) ou (II').

La méthode de l'invention permet d'identifier une transformation chimique énantio- ou stéréo-sélective dans un échantillon.

Avantageusement, la transformation chimique est effectuée au moyen d'un catalyseur et en particulier d'une enzyme. A titre d'exemple non limitatif, la méthode de l'invention permet la détection de l'activité d'une enzyme choisie parmi : Lipase, Estérase, Protéase, Glycosidase, Glycosyl transférasse, Phosphatase, Kinase, Mono ou Dioxygenase, Haloperoxidase, Lignine peroxydase, Diarylpropane peroxydase, Epoxyde hydrolase, Nitrile hydratase, Nitrilase, Transaminase, Amidase, Acylase, Dihydroxylase, Phytase, Xylanase, Nucléase, Réductase.

Dans le cas d'une transformation chimique spontanée ou non, on peut citer notamment: une hydrolyse spontanée ou thermique d'un ester, une dihydroxylation d'oléfine par les réactifs AD-mix, une hydrolyse des époxydes par des complexes de Chrome.

**La présente décrit** enfin un procédé de détection et/ou de quantification d'une transformation chimique connue, dans un échantillon consistant à mettre en oeuvre la méthode de détection d'une transformation chimique décrite précédemment en présence dudit échantillon et d'un substrat adapté à l'activité recherchée.

L'invention permet également de détecter le substrat de formule (I) ou (I') en présence d'un réactif catalyseur chimique ou biochimique.

La méthode de l'invention peut être utilisée pour le criblage, notamment à haut débit, d'un catalyseur à partir d'une banque d'expression in *vivo* ou in *vitro.* Ces banques peuvent par exemple être préparées à partir de microorganismes ou de microalgues présentant préférentiellement des propriétés extrêmophiles.

La méthode de l'invention peut également être utilisée pour identifier des catalyseurs présentant une activité différente par rapport à une activité de départ. Ces catalyseurs seront par exemple des produits de mutagénèse dirigée ou d'évolution dirigée.

Une forme particulière de mise en oeuvre de la méthode de l'invention concerne le cas où le substrat de formule (I) ou (I') est lui-même le produit d'une première transformation chimique inconnue, dont on souhaite identifier l'activité.

A titre d'exemple de cette forme particulière de mise en oeuvre du procédé de l'invention, on peut citer la réaction d'époxydation d'oléfines pour les transformer en époxydes de formules (I) ou (I') ou (VII), lesdits epoxydes subissent ensuite une réaction enzymatique afin d'obtenir les diols de formules (II) ou (II') ou (X), lesquels seront soumis à une réaction d'oxydation chimique par le periodate.

La méthode de l'invention est remarquable en ce qu'elle permet également l'identification et l'isolement de nouveaux catalyseurs chimiques ou biochimiques susceptibles de transformer un substrat de formule (I) ou (I') respectivement en un composé de formule (II) ou (II'). En effet la méthode de l'invention permet de révéler la présence d'un catalyseur qui, grâce à l'oxydation chimique, génère un signal directement ou indirectement détectable.

En conséquence, l'invention a également pour objet l'utilisation de la méthode de détection d'une transformation chimique, comme décrite précédemment, pour l'identification de nouveaux catalyseurs, ou de l'activité nouvelle d'un catalyseur connu. L'invention se rapporte aussi à un catalyseur susceptible d'être identifié par la méthode de l'invention.

Ces nouveaux catalyseurs sont spécifiques des conditions expérimentales utilisées lors de leur détection, ils correspondent avantageusement à des enzymes.

Ces nouvelles enzymes sont spécifiques des conditions expérimentales utilisées lors de leur détection.

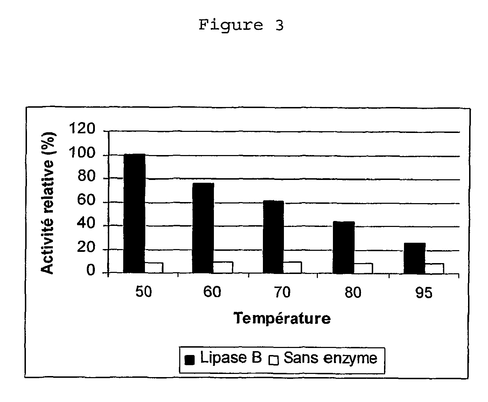

D'autres avantages et caractéristiques de l'invention apparaîtront à la lecture des exemples ci-dessous qui font partie des travaux expérimentaux effectués par la Demanderesse dans le cadre de l'invention et où l'on fait référence aux figures en annexe dans lesquelles
- La figure 3 représente l'activité enzymatique de la lipase de *Candida antarctica* en fonction de la température. Les réactions enzymatiques ont été réalisées dans le tampon PIPES (0.1 M pH 7) à 50, 60, 70, 80 et 95°C pendant 40 minutes en utilisant le 2-hydroxy-4-p-nitrophényl-butyl-decanoate. (■) lipase de *Candida antarctica;* (□) sans enzyme.

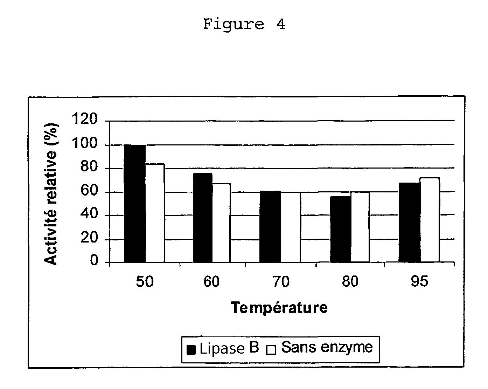

- La figure 4 illustre l'activité enzymatique de la lipase de *Candida antarctica* en fonction de la température. Les réactions enzymatiques ont été réalisées dans le tampon PIPES (0.1 M pH 7) à 50, 60, 70, 80 et 95°C pendant 40 minutes en utilisant le para-nitrophénylbutyrate. (■) lipase de *Candida antarctica;* (□) sans enzyme.

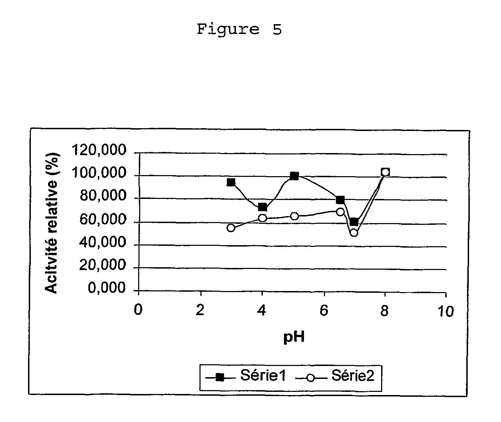

- La figure 5 illustre l'activité de la lipase de *Candida antarctica* en fonction du pH de la solution. Les tests ont été réalisés en utilisant le 2-hydroxy-4-p-nitrophényl-butyl-decanoate.

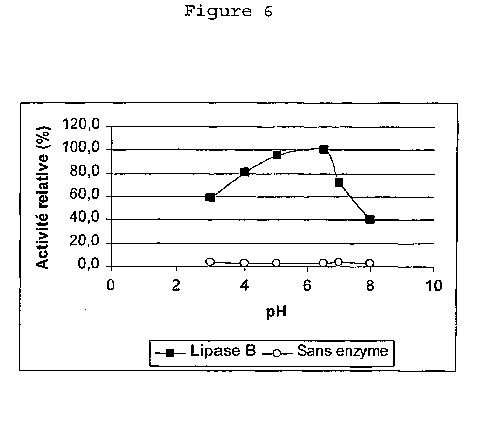

- la figure 6 illustre l'activité de la lipase de *Candida antarctica* en fonction du pH de la solution. Les tests ont été réalisés en utilisant le para-nitrophénolbutyrate.

### I - Synthèse des substrats.

### Exemple 1: Préparation du 4-(7-coumarinyloxy)-1-butène.

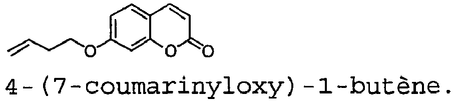

Une suspension de 7-hydroxycoumarine (3 g, 19.6 mmol) dans le DMF (20 mL) est traitée avec du NaH (60% suspension dans l'huile, 1.03 g, 25.9 mmol), puis avec du 4-bromo-1-butène (2.65 g, 19.6 mmol. On chauffe à 60 °C pendant 24 h, puis on dilue avec de l'acétate d'éthyle (400 mL) et on lave avec de l'eau (3 x 300 mL) puis avec du NaOH 1 M (3 x 200 mL). On chromatographie le résidu (acétate d'ethyle-hexane) pour obtenir le 4-(7-coumarinyloxy)-1-butène (2.7 g, 67 %).

### Exemple 2: Préparation du (S)-4-(7-coumarinyloxy)-1,2-butanediol.

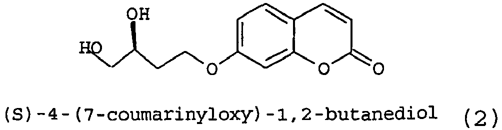

On agite une suspension d'AD-mix-alpha (4.2 g) dans 30 mL de t-BuOH/eau 1:1 jusqu'à l'obtention d'une seule phase (5 min.). On refroidit alors à 0°C et on ajoute l'oléfine (0.648 g, 3 mmol). Après 16 h à 0 °C, on quenche avec du Na₂S₂O₅ (4.5 g). Après une heure à température ambiante, on extrait avec du CH₂Cl₂ et on chromatographie (élution CH₂Cl₂/acétone 7:3) pour obtenir 0.6 g (80 %) de diol pur (S).

m.p. 92-93°C, [a]_{D} ²⁰=-22.4 (c 0.46, CH₃OH); ¹H-NMR (CD₃OD) _ 7.83 (d, 1H, *J*=9.8 Hz), 7.47 (d, 1H, *J*=8.3 Hz), 6.85-6.92 (m, 2H), 6.21 (d, 1H, J=9.3 Hz), 4.16-4.23 (m, 2H), 3.81-3.93 (m, 1H), 3.54 (d, 2H, J=6.35 Hz), 1.97-2.13 (m, 1H), 1.74-1.91 (m, 1H).

La même procédure avec l'AD-mix béta donne l'énantiomère R.

### Exemple 3: Préparation du (S)-4-(7-coumarinyloxy) - 1,2-epoxybutane.

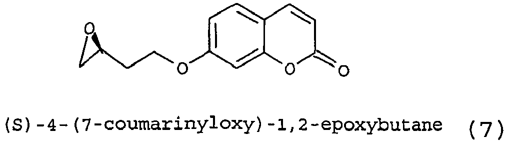

A une solution de (S)-4-(7-coumarinyloxy)-1,2-butanediol (0.15 g, 0.6 mmmol) dans le CH₂Cl₂, on ajoute du triméthyl orthoacétate (1 mL), puis du pyridinium paratoluène sulfonate (1 mg). Après 40 min., on évapore le tout et on dissout le résidu dans le CH₂Cl₂ (1 mL) et on traite avec Me₃SiCl (0.1 mL, 0.78 mmol). Après une heure, on ajoute du méthanol sec (1 mL) et du carbonate de potassium (0,2 g, 1.5 mmol). On laisse 2 h à 20 °C, puis on filtre et on chromatographie le filtrat (hexane/acétate d'éthyle 6:4) pour obtenir l'époxyde (S) (0.097 g, rendement 70%).

m.p. 61-64°C, [α]_{D} ²⁰ =-23.0 (*c* 0.3, CHCl₃) ; ¹H-NMR (CDCl₃) : 7.58 (d, 1H, *J*=9.3 Hz), 7.31 (d, 1H, *J*=8.3 Hz), 6.75-6.81 (m, 2H), 6.18 (d, 1H, *J*=9.8 Hz), 4.03-4.19 (m, 2H), 3.05-3.14 (m, 1H), 2.78 (t, 1H, *J*=4.9 Hz), 2.53 (dd, 1H, *J*=4.9 et 2.4 Hz), 2.04-2.20 (m, 1H), 1.79-1.97 (m, 1H).

La même procédure avec le (R)-4-(7-coumarinyloxy)-1,2-butanediol donne l'énantiomère R.

### Exemple 4: Préparation du (S)-1,2-Diacetoxy-4-(7-coumarinyloxy)-butane.

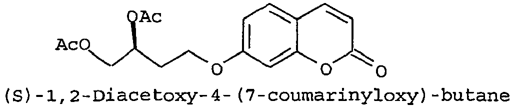

A une solution de (S)-4-(7-coumarinyloxy)-1,2-butanediol (0.1 g, 0.4 mmol) dans 4 mL de pyridine anhydre à 0°C, on ajoute 2 mL d'anhydride acétique. Après 18h à 20 °C, on évapore avec du toluène, et on chromatographie le résidu pour obtenir le diacétate S (rendement quantitatif).

m.p. 74-76 °C, [α]_{D}²⁰=-9.8 (*c* 0.5, CHCl₃) ; ¹H-NMR (CDCl₃) : 7.61 (d, 1H, *J*=9.8 Hz), 7.34 (d, 1H, *J*=8.8 Hz), 6.74-6.82 (m, 2H), 6.22 (d, 1H, *J*=9.3 Hz), 5.24-5.35 (m, 1H), 4.32 (dd, 1H, *J*=11.7 et 3.4 Hz), 3.97-4.15 (m, 2H), 4.10 (dd, 1H, *J*=11.7 et 5.9 Hz), 2.05-2.16 (m, 2H), 2.05 (s, 6H).

La même procédure avec le (R)-4-(7-coumarinyloxy)-1,2-butanediol donne l'énantiomère R.

### Exemple 5 : Préparation du (S)-1-Acetoxy-4-(7-coumarinyloxy)-2-butanol.

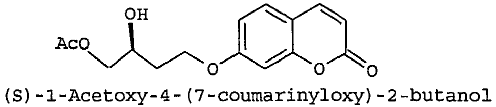

A une solution de (S)-4-(7-coumarinyloxy)-1,2-butanediol (0.090 g, 0.36 mmol) et de triéthylamine (0.1 mL, 0.72 mmol) dans 8 mL de CH₂Cl₂ sec, on ajoute du chlorure d'acétyle (0.36 mmol, 0.024 mL). Après 40 min. à 0°C, on dilue avec du CH₂Cl₂, on lave avec une solution aqueuse de NaHCO₃. La chromatographie du résidu après évaporation donne le monoacétate S (0.058 g, 0.22 mmol, 60 %).

[α]_{D}²⁰=-9.6 (c 0.31, CHCl₃) ; ¹H-NMR (CDCl₃) : 7.61 (d, 1H, *J*=9.8 Hz), 7.33 (d, 1H, *J*=7.8 Hz), 6.78-6.83 (m, 2H), 6.21 (d, 1H, *J*=8.8 Hz), 4.01-4.29 (m, 5H), 2.09 (s, 3H), 1.91-2.00 (m, 2H).

La même procédure avec le (R)-4-(7-coumarinyloxy)-1,2-butanediol donne l'énantiomère R.

### Exemple 6 : Préparation du 1-Amino -4-(7-coumarinyloxy)-2-butanol.

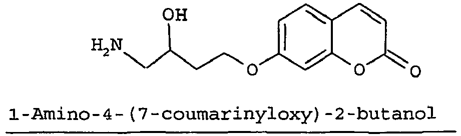

A une solution de 4-(7-coumarinyloxy)-1,2-epoxybutane racémique (0.150 g, 0.65 mmol) dans 2 mL d'éthanol, on ajoute 5 mL d'ammoniaque 30 % aqueux et du Gd(OTf₃) (0.039 g, 0.065 mmol). On chauffe à 65 °C pendant 15 h, on évapore le solvant, on dilue le résidu dans l'acétate d'éthyle et on lave avec une solution saturée en NaCl. Le résidu donne l'amine brute (0.081 g, rendement 50% ) qu'on utilise sans purification pour l'étape suivante.

### Exemple 7 : Préparation du 1-Phenylacetamido-4-(7-coumarinyloxy)-2-butanol.

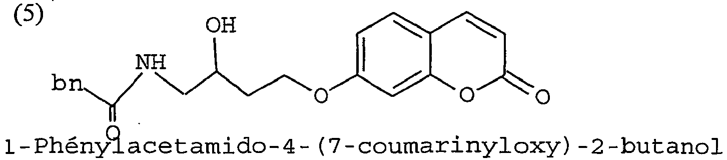

A une solution de 1-Amino -4-(7-coumarinyloxy)-2-butanol (0.162 g, 0.65 mmol) dans 3 mL de CH₂Cl₂ sec à 0°C, on ajoute de la triéthylamine (1.3 mmol, 0.18 mL) puis du chlorure de phénotype (0.65 mmol, 0.085 mL). On agite à 0°C pendant 2 h, puis on dilue avec du CH₂Cl₂ et on lave avec une solution aqueuse saturée de NaHCO₃. On chromatographie le produit brut pour obtenir l'amide (0.170 g, 0.48 mmol, 74 %).

Exemple 8: Préparation du 6-methoxy-2-naphthaldéhyde et 6-dimethylamino-2-naphthaldéhyde.

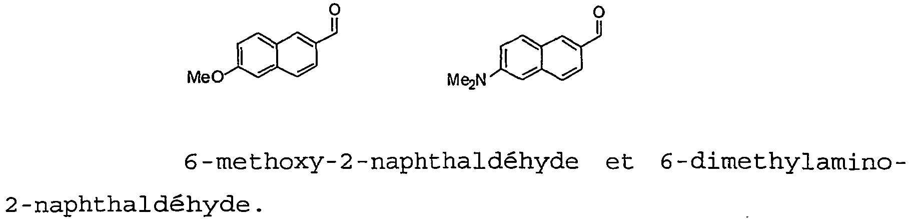

On prépare le 6-methoxy-2-naphthaldéhyde par traitement successif du 2-bromo-6-methoxynaphthalène en solution dans l'éther sec avec n-butyl lithium puis du diméthyl formamide. (Littérature: J. Med. Chem. 1998, 1308-1312). Le 6-dimethylamino-2-naphthaldehyde est préparé selon la procédure décrite (Barbas et al., Proc. Natl. Acad. Sci. USA 1998, 95, 15351) comme suit: on introduit de la diméthylamine gazeuse dans un mélange de 2.4 mL de benzène sec et 2.4 mL d'hexaméthylphosphoramide (HMPA) jusqu'à la dissolution de 750 mg (16.7 mmol). A 0°C et sous atmosphère inerte, on ajoute du n-butyl lithium (1.6 M dans l'hexane, 16.7 mmol) puis après 15 minutes le 6-methoxy-naphthaldehyde (390 mg, 2.09 mmol). On laisse sous agitation pendant 14 heures à 20 °C, puis on verse dans du tampon phosphate aqueux pH 7.4 et on extrait à l'éther. La purification par chromatographie donne le 6-dimethylamino-2-naphthaldéhyde (350 mg, 84 %) .

6-methoxy-2-naphthaldéhyde: ¹H-NMR (CDCl₃) : 10.09 (s, 1H), 8.26 (s, 1H), 7.78-7.95 (m, 3H), 7.17-7.26 (m, 2H), 3.96 (s, 3H).

### Exemple 9 : Préparation du 6-méthoxy et du 6-dimethylamino-2-vinylnaphthalene.

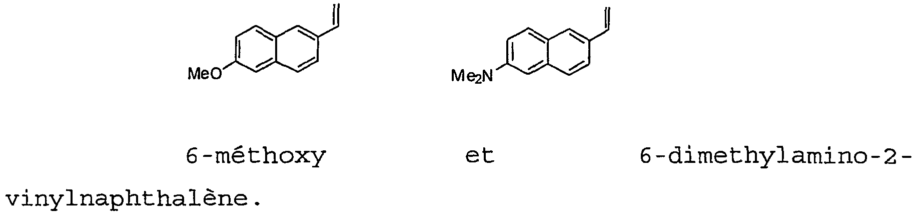

A une solution d'ylure instantané (Ph₃P⁺Br⁻ + NaNH₂, 1.48 g, 2.4 mmol) dans du THF anhydre (5 ml), on ajoute le 6-méthoxy-naphthaldéhyde (432 mg, 2.35 mmol). Après une heure sous agitation à température ambiante, on dilue avec de l'éther, on filtre sur célite, et on chromatographie (hexane/AcOEt 5:1) pour obtenir le 6-méthoxy-2-vinyl-naphthalène (390 mg, 90 %).

6-méthoxy-2-vinyl-naphthaldéhyde: ¹H-NMR (CDCl₃) : 7.59-7.74 (m, 4H), 7.11-7.16 (m, 2H), 6.86 (dd, 1H, J= 17.3 et 10.9 Hz), 5.82 (d, 1H, J=17.6 Hz), 5.28 (d, 1H, J= 10.7 Hz), 3. 93 (s, 3H).

### Exemple 10 : Préparation du (R) et du (S) 6-méthoxy-2-(1',2'-dihydroxyéthyl)naphthalène.

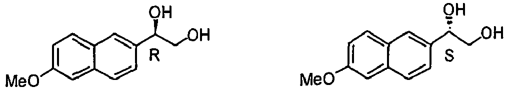

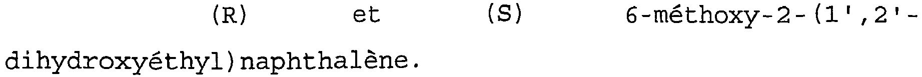

Ces produits, sous forme d'énantiomères R (>99% ee) et S (>99% ee), ont été préparés par dihydroxylation asymétrique de Shamisens de l'oléfine correspondante comme décrit plus haut pour les dérivés de coumarine.

Diol: ¹H-NMR (Acétone-d6) : 7.71-7.84 (m, 3H), 7.52 (dd, 1H, J=8.5 et 1.5 Hz), 7.25-7.31 (m, 1H), 7.16 (dd, 1H, J= 8.9 et 2.5), 4.87 (dd, 1H, J=7.5 et 4.2 Hz), 3.94 (s, 3H), 3.50-3.80 (m, 4H, CH₂O+2 OH).

### Exemple 11 : Préparation du (R) - et du (S)-2-(6-méthoxy-2-naphthyl)oxyrane.

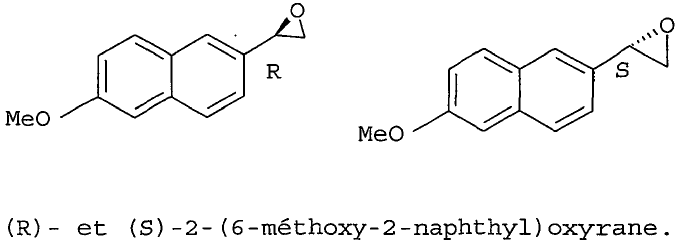

Ces produits ont été préparés à partir des diols correspondants selon la méthode décrite plus haut pour les dérivés de coumarine et obtenus optiquement purs (ee >99% selon HPLC chirale)

¹H NMR (CDCl3) : 7.70-7.75 (m, 3H), 7.26-7.32 (m, 1H), 7.13-7.19 (m, 2H), 4.00 (dd, 1H, J= 4.1 et 2.4 Hz), 3.92 (s, 3H), 3.22 (dd, 1H, J= 5.4 et 4.0 Hz), 2.91 (dd, 1H, J= 5.4 et 2.4 Hz).

### II - Oxydation par le periodate.

L'oxydation de 1,2 diols et d'amino-alcools par le periodate dans un milieux aqueux est une réaction bien connue de l'homme du métier. Cette réaction apporte une alternative purement chimique pour la conversion d'alcools en groupes carbonyles en milieu aqueux avec une chimioselectivitée élevée en présence d'autres groupes fonctionnels. La Demanderesse a trouvé maintenant que, avantageusement, des enzymes hydrolytiques qui libèrent des diols ou des aminoalcools à partir de substrats résistants à l'oxydation par le periodate sont détectables par fluorescence en présence de periodate et d'albumine de sérum bovine (BSA).

Les diesters (R)- (S)- ainsi que les monoesters (R)- et (S)- représentés par la formule (I) (composés 1 a/b, 3 a/b) constituent d'excellents substrats pour les lipases et les estérases étant donné leur similarité structurelle avec les glycérides. Les amides représentées par la formule I (composé 5) réagissent avec les amidases et les peptidases. Les époxydes (R)- et (S)- représentés par la formule (II) (composés 7 et 8) réagissent avec les enzymes époxy-hydrolases. Ainsi, la demanderesse a trouvé que la transformation subie par ces substrats fluorogéniques transformés par le periodate de sodium, en présence des enzymes hydrolytiques fournit une nouvelle méthode de détection desdites enzymes.

### III - Stabilité des substrats.

Les substrats acétates 1, l'amide 5 et l'époxyde 6 sont stables en présence de periodate et de BSA sans les milieux aqueux. Dans les mêmes conditions, le diol 2 et l'amino-alcool 6 libèrent l'umbélliférone. Dans des conditions de réaction optimisées utilisant 100*µ*m de substrat, 1mM de periodate de sodium, 2 mg/ml de BSA dans un tampon borate à pH 8,8, le diol 2 est transformé en umbélliférone avec un rendement de 72% sans réactions secondaires observables. Le rendement de l'oxydation est indépendant de la présence de BSA, ce qui indique que ces réactifs n'interfèrent de manière notoire entre eux. L'oxydation de l'amino-alcool 6 se produit avec un rendement de 85% dans les mêmes conditions.

### IV - Développement de l'essai.

Les acétates 1, l'amide 5 et les époxydes (R)-7 et (S)-7 ont été testés en présence des enzymes hydrolytiques correspondantes. Les acétates ont été testés vis-à-vis de 25 estérases et lipases différentes. Les époxydes ont été testés vis-à-vis de l'époxy-hydrolase extraite d'Aspergillus Niger (X-J. Chen, A. Archelas, R. Furstoss, J.Org.Chem. 1993, 58 , 5528) et de Rhodotorula glutinis (C.A.G.M. Weijers, A.L. Botes, M.S. van Dyk, J.A.M. de Bont, Tetrahedron Asymm. 1998,9, 467).

La phenylacétamide 5 a été testée vis-à-vis de la penicilline G acylase, de la chimitripsine et de la papaïne. Une augmentation de la fluorescence clairement dépendante du temps est observée lorsque l'activité enzymatique est présente. Les analyses effectuées par chromatographie liquide de haute pression (HPLC) confirment que l'augmentation de la fluorescence est provoquée par la libération de l'umbélliférone des substrats.

Dans tous les cas les résultats obtenus sont similaires tant à pH 7,2 qu'à pH 8,8.

Toutes les enzymes qui ont montré une activité à l'encontre d'un quelconque substrat conservent leur activité que ce soit en effectuant une preincubation de l'enzyme avec le substrat, avant l'addition du periodate et de la BSA ou lors de la réaction, en présente de tous les composants. Ceci prouve clairement que l'addition du periodate et de la BSA en tant qu'agents secondaires n'affectent pas l'activité enzymatique des enzymes qui doivent être analysées. Les analyses par HPLC de la réaction avec les enzymes actives en absence de periodate et de BSA montrent que le diol 2 et l'amino-alcool 6 sont réellement libérées par les enzymes ayant été considérées comme actives dans le test de fluorescence.

### V - Mesure de l'activité lipase en utilisant les substrats 2-hydroxy-4-p-nitrophényl-butyl-decanoate et p-nitro-phénylbutyrate

L'activité hydrolytique de la lipase de *Candida antarctica* a été testée sur 2 substrats: 2-hydroxy-4-p-nitrophényl-butyl-decanoate et p-nitro-phénylbutyrate. L'activité lipasique est détectée après hydrolyse des fonctions esters des substrats conduisant à la libération (directe ou indirecte) du para-nitrophénol de couleur jaune qui est mesurée par colorimétrie à 414 nm. Les tests ont été réalisées à différents températures et pH.

Les solutions mères du 2-hydroxy-4-p-nitrophényl-butyl-decanoate et du para-nitrophénolbutyrate (SIGMA) sont préparées à la concentration de 20 mM dans respectivement de l'acétonitrile et de l'isopropanol. La concentration de lipase B utilisée est de 0.01 mg/ml dans un tampon phosphate (100 mM pH 7).

### V.1 - Mesure de l'activité lipase en fonction de la température

### V.1.1 - Dosage de l'activité lipase avec le 2-hydroxy-4-p-nitrophényl-butyl-decanoate.

A 13.6 µmol de 2-hydroxy-4-p-nitrophényl-butyl-decanoate sont ajoutés 74 µl de tampon PIPES (100mM pH 7) et 10 µl d'une solution de lipase à 0.01 mg/ml. La réaction est laissée au bain-marie pendant 40 minutes à 50, 60, 70, 80 et 95°C. Les échantillons sont ensuite retirés du bain-marie. Puis 40 µl de NaIO₄ (100 mM) et 4 µl de BSA (50 mg/ml) sont ajoutés dans le mélange. Le pH de la solution est ensuite ajusté à pH 10 en ajoutant 40 µl d'une solution de Na₂CO₃ à 0.2 M. Le para-nitrophénol libéré est ensuite mesuré au colorimètre à 414 nm.

Un témoin négatif sans enzyme a été réalisé dans les mêmes conditions expérimentales.

### V.1.2 - Dosage de l'activité lipase avec le p-.nitrophénylbutyrate

A 13.6 µmol de para-nitrophénylbutyrate sont ajoutés 74 µl de tampon PIPES (100mM pH 7) et 10 µl d'une solution de lipase à 0.01 mg/ml. La réaction est laissée au bain-marie pendant 40 minutes à 50, 60, 70, 80 et 95°C. Les échantillons sont ensuite retirés du bain-marie et le pH de la solution est ajusté à pH 10 avec une solution de Na₂CO₃ 0.2 M. Le para-nitrophénol libéré est ensuite mesuré au colorimètre à 414 nm.

Un témoin négatif sans enzyme a été réalisé dans les mêmes conditions expérimentales

### V.2. - Mesure de l'activité lipase en fonction du pH de la solution

L'activité enzymatique de la lipase de *Candida antarctica* a été réalisée à différents pH en utilisant les tampons (100 mM) listés dans le tableau VII ci-dessous.

**Tableau VII**

| Tampon | PH |
|---|---|
| Formate | 3 |
| Formate | 4 |
| Acétate | 5 |
| MES | 6,5 |
| PIPES | 7 |
| phosphate | 8 |

### V.2.1 - Dosage de l'activité lipase avec le 2-hydroxy-4-p-nitrophényl-butyl-decanoate

A 13.6 µmol de 2-hydroxy-4-p-nitrophényl-butyl-decanoate sont ajoutés 74 µl de tampon au pH désiré (100mM) et 10 µl d'une solution de lipase à 0.01 mg/ml. La réaction est laissée au bain-marie pendant 40 minutes à 60°C. Les échantillons sont ensuite retirés du bain-marie. Puis 40 µl de NaIO₄ (100 mM) et 4 µl de BSA (50 mg/ml) sont ajoutés dans le mélange. Le pH de la solution est ensuite ajusté à pH 10 en ajoutant 40 µl d'une solution de Na₂CO₃ à 0.2 M. Le para-nitrophénol libéré est ensuite mesuré au colorimètre à 414 nm.

Un témoin négatif sans enzyme a été réalisé dans les mêmes conditions expérimentales.

### V.2.2 Dosage de l'activité lipase avec le p-nitrophénylbutyrate

A 13.6 µmol de para-nitrophénylbutyrate sont ajoutés 74 µl de tampon au pH désiré (100mM) et 10 µl d'une solution de lipase à 0.01 mg/ml. La réaction est laissée au bain-marie pendant 40 minutes à 60°C. Les échantillons sont ensuite retirés du bain-marie et le pH de la solution est ajusté à pH 10 avec une solution de Na₂CO₃ 0.2 M. Le para-nitrophénol libéré est ensuite mesuré au colorimètre à 414 nm.

Un témoin négatif sans enzyme a été réalisé dans les mêmes conditions expérimentales.

### V.3 - Résultats de la détection de l'activité lipase.

Le 2-hydroxy-4-p-nitrophényl-butyl-decanoate et le substrat commercial para-nitrophénylbutyrate ont été testés vis à vis de la lipase de *Candida antarctica.* Une apparition de la coloration jaune liée à la libération du para-nitrophénol est observée lorsque l'activité hydrolytique est présente.

L'activité de la lipase en fonction de la température d'incubation a été mesurée en utilisant le 2-hydroxy-4-p-nitrophényl-butyl-decanoate et le substrat commercial para-nitrophénylbutyrate. Le tableau VIII montre les mesures de l'activité de la lipase de *Candida antarctica* effectuées en utilisant le 2-hydroxy-4-p-nitrophényl-butyl-decanoate et le para-nitrophénylbutyrate. Les activités sont exprimées en %. Les réactions enzymatiques ont été réalisées dans le tampon PIPES (0.1 M pH 7) à 50, 60, 70, 80 et 95°C pendant 40 minutes. Un contrôle négatif sans enzyme a été également réalisé en utilisant les mêmes conditions expérimentales.

**Tableau VIII**

| | 2-hydroxy-4-p-nitrophényl-butyl-decanoate | | p-nitrophénylbutyrate | |
|---|---|---|---|---|
| Température (°C) | Lipase B | Sans enzyme | Lipase B | Sans enzyme |
| 50 | 100 | 8,7 | 100 | 83,6 |
| 60 | 75,9 | 9,4 | 75,4 | 67,3 |
| 70 | 61,5 | 9,5 | 60,4 | 59 ,5 |
| 80 | 44,2 | 8,1 | 55,9 | 59,8 |
| 95 | 26,1 | 8,6 | 66,7 | 71,5 |

L'activité hydrolytique de la lipase en fonction du pH de la solution a été également dosée. Le tableau IX ci-dessous montre l'activité de la lipase de *Candida antarctica* mésurée en utilisant le 2-hydroxy-4-p-nitrophényl-butyl-decanoate et le para-nitrophénylbutyrate. Les activités sont exprimées en %. Les réactions enzymatiques ont été incubées à 60°C pendant 40 minutes à pH 3, 4, 5, 6.5, 7 et 8. Un contrôle négatif sans enzyme a été également réalisé en utilisant les mêmes conditions expérimentales.

**Tableau IX**

| | 2-hydroxy-4-p-nitrophényl-butyl-decanoate | | p-nitrophénylbutyrate | |
|---|---|---|---|---|
| pH | Lipase B | Sans enzyme | Lipase B | Sans enzyme |
| 3 | 58,9 | 3,9 | 94,2 | 54,9 |
| 4 | 80,9 | 2,7 | 73 | 63,6 |
| 5 | 96,1 | 2,6 | 100 | 65,6 |
| 6,5 | 100 | 2,9 | 79,5 | 69,2 |
| 7 | 72 | 3,4 | 60,8 | 51,4 |
| 8 | 40 | 2,8 | 103,9 | 103,9 |

### VI. Détection de la libération d'un composé volatile.

Les premiers tests ont été menés avec trois substrats différents: le benzaldéhyde, la forme volatile, l'hydrobenzoïne et le 1,2-pentanediol, les formes vicinales.

10mg de chaque produit sont mélangés avec 10mg de NaIO4 et 1g de MgS04, puis laissés simplement à l'air sur une étagère.

La détection des arômes est réalisée de temps en temps en prélevant un peu de poudre et en ajoutant de l'eau. Les résultats correspondant aux observations relatives à la perception des parfums sont résumés dans le Tableau X ci-dessous

**Tableau X**

| Durée | Benzaldéhyde | Hydrobenzoïne | 1,2-Pentanediol |
|---|---|---|---|
| 1 jour | odeur forte | une simple agitation du solide aux ultrasons suffit à libérer une odeur perceptible | une simple agitation du solide aux ultrasons suffit à libérer une odeur perceptible |
| 5 jours | plus d'odeur | 2 ou 3 gouttes d'eau doivent être ajoutées à quelques milligrammes de solide | 2 ou 3 gouttes d'eau doivent être ajoutées à quelques milligrammes de solide |
| 1 mois | idem | 2 ou 3 ml d'eau doivent être ajoutés à quelques milligrammes de solide | 2 ou 3 ml d'eau doivent être ajoutés à quelques milligrammes de solide |
| 5 mois | idem | idem | Une plus grande quantité de solide est nécessaire |

La détection de l'odeur « amande » du benzaldéhyde, la forme volatile, est stable au moins un jour. En revanche, grâce au procédé de l'invention, l'oxydation des diols vicinaux correspondant au benzaldéhyde permet de diffuser pendant plusieurs mois l'odeur « amande du benzaldéhyde.

### Annexe

**Tableau XI**

| Liste des substrats par groupements fonctionnels | |
|---|---|
| ***Diols, amino-alcools et azido-alcool*** | |
| 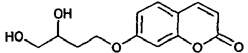 | 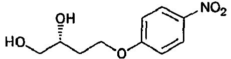 |
| 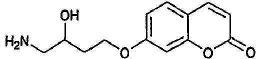 | 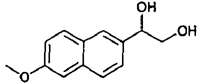 |
| 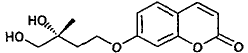 | 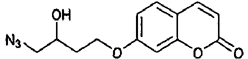 |
| 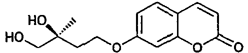 | 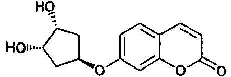 |
| 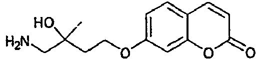 | 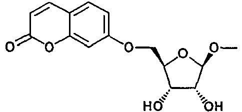 |
| 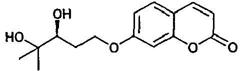 | 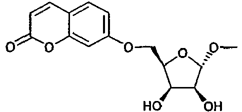 |
| 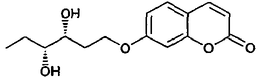 | 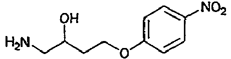 |
| 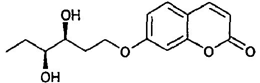 | 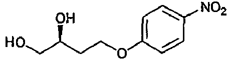 |
| 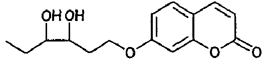 | 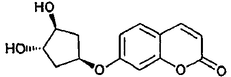 |
| 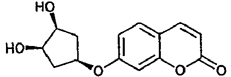 | |

| **Esters** | |
|---|---|
| 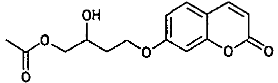 | 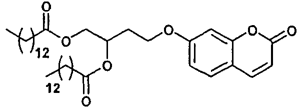 |
| 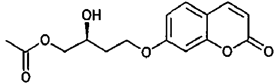 | 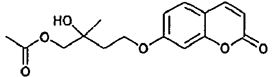 |
| 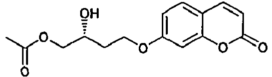 | 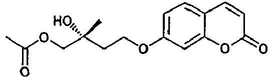 |
| 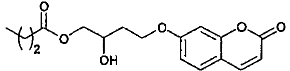 | 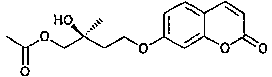 |
| 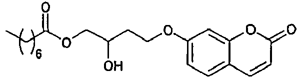 | 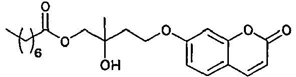 |
| 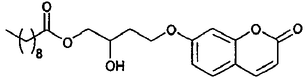 | 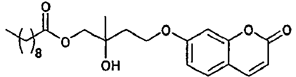 |
| 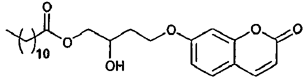 | 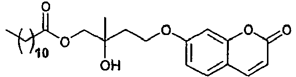 |
| 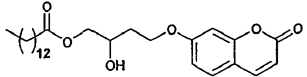 | 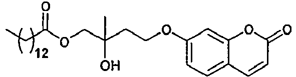 |
| 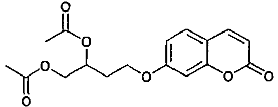 | 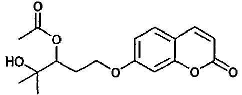 |
| 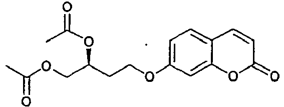 | 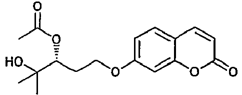 |
| 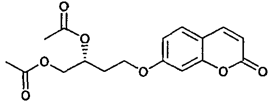 | 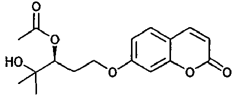 |
| 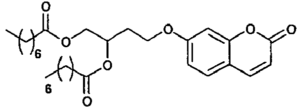 | 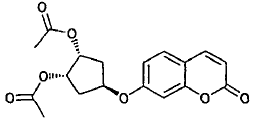 |
| 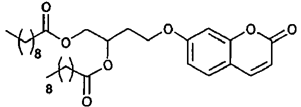 | 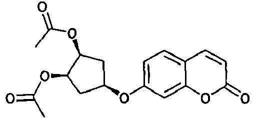 |
| 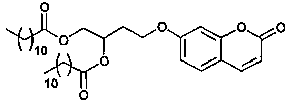 | 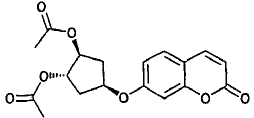 |
| 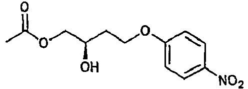 | 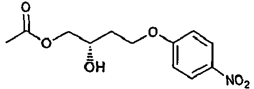 |
| 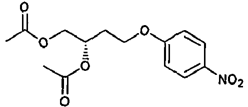 | 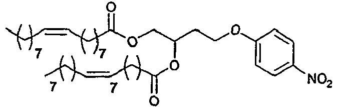 |
| 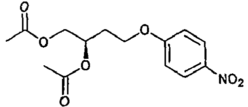 | 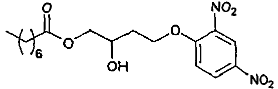 |
| 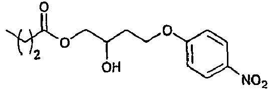 | 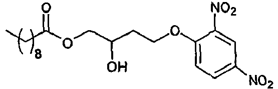 |
| 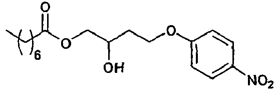 | 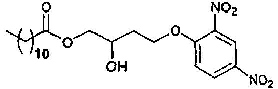 |
| 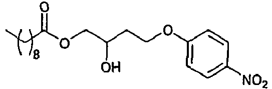 | 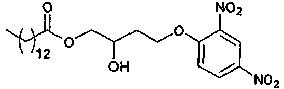 |
| 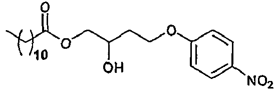 | 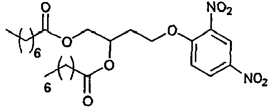 |
| 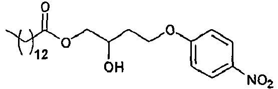 | 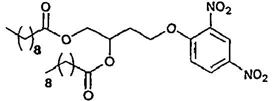 |
| 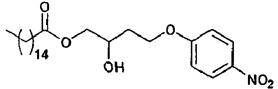 | 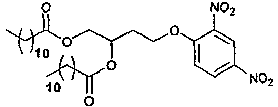 |
| 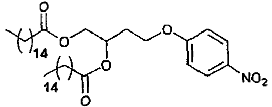 | 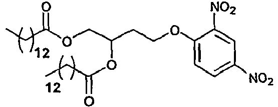 |
| 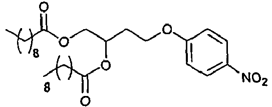 | 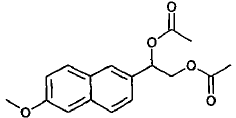 |
| 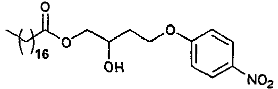 | 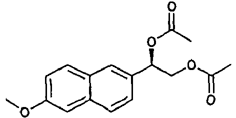 |
| 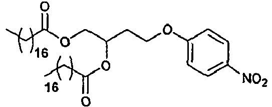 | 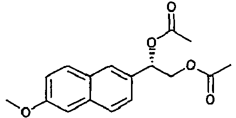 |
| 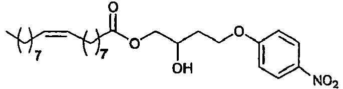 | 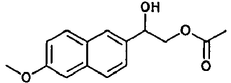 |
| 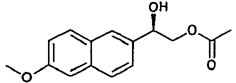 | 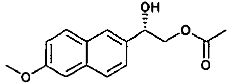 |
| 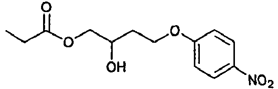 | 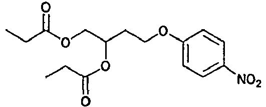 |

| ***Amides*** | |
|---|---|
| 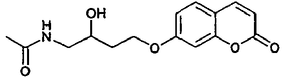 | 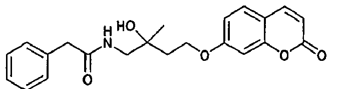 |
| 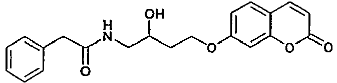 | 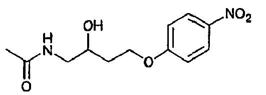 |
| 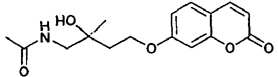 | 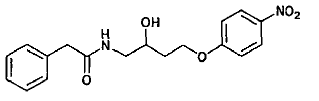 |

| ***Alcools*** | |
|---|---|
| 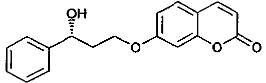 | 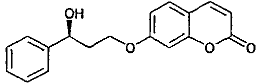 |

| ***Phosphates*** | |
|---|---|
| 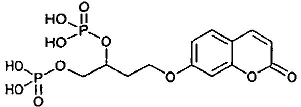 | 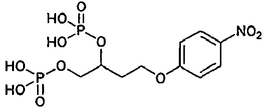 |

| ***Triols et derivés*** | |
|---|---|
| 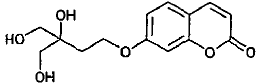 | 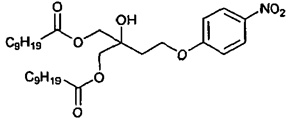 |
| 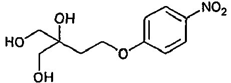 | 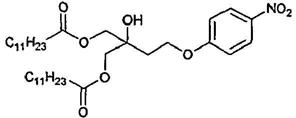 |
| 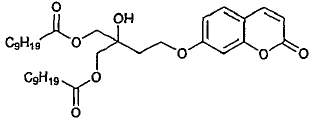 | 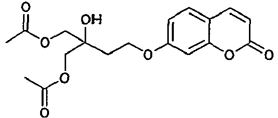 |
| 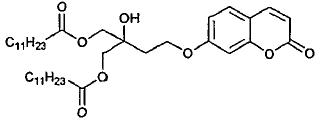 | 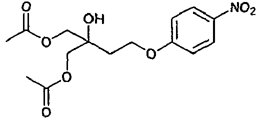 |

| **Epoxides** | |
|---|---|
| 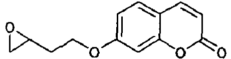 | 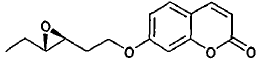 |
| 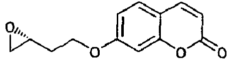 | 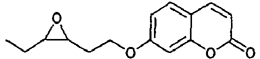 |
| 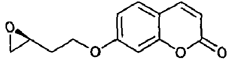 | 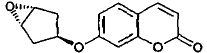 |
| 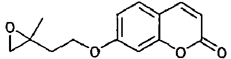 | 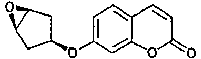 |
| 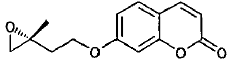 | 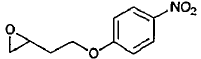 |
| 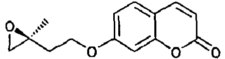 | 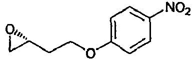 |
| 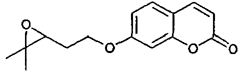 | 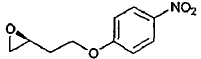 |
| 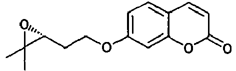 | 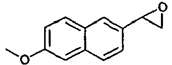 |
| 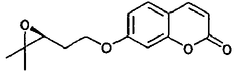 | 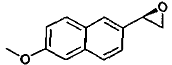 |
| 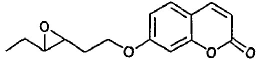 | 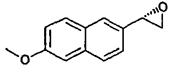 |
| 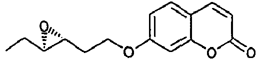 | |

| **Carbonates** | |
|---|---|
| 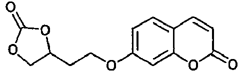 | 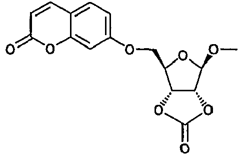 |
| 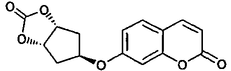 | 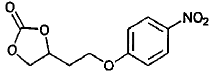 |
| 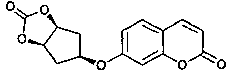 | 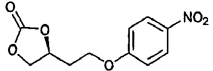 |
| 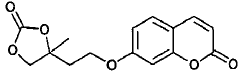 | 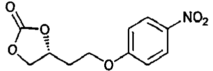 |
| 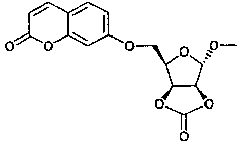 | |

## Revendications

1. Méthode de détection de la transformation chimique et/ou enzymatique d'un substrat en un composé détectable, **caractérisée en ce qu'**elle comprend les étapes suivantes :
a) la transformation chimique d'un substrat de formule (I) dans laquelle la liaison C₁-C₂ est insensible à une coupure par une réaction d'oxydation chimique : 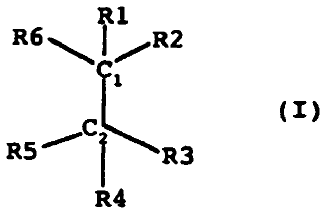 en un composé de formule (II) dans laquelle la liaison C₁-C₂ est sensible à une coupure par une réaction d'oxydation chimique effectuée au moyen d'un agent chimique oxydant : 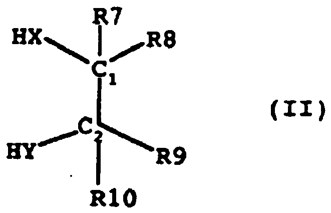 et,
(b) l'oxydation chimique du composé de formule (II) obtenu à l'étape (a) qui coupe la liaison C₁-C₂ pour obtenir directement ou indirectement un produit détectable,
et **en ce que** dans les composés de formules (I) et (II) :
- au moins un des groupes R₁ à R₆ est un groupement Di qui est insensible à la réaction d'oxydation de l'étape(b) et qui présente des propriétés détectables après la coupure de la liaison C₁-C₂,
- les groupes R₁ à R₆, identiques ou différents, sont choisis parmi un atome d'hydrogène, un groupe alkyle substitué ou non, un groupe fonctionnel substitué ou non et dans le cas d'un groupe fonctionnel de formules -OR₁₂, -SR₁₂ ,-NR₁₁R₁₂, R₁₁ est choisi parmi un atome d'hydrogène, un groupe alkyle, un groupe aryle, substitués ou non, et R₁₂ n'est pas un atome d'hydrogène,
- le ou les groupes R₁ à R₆ formant après l'étape (a) les groupes de formules -XH et -YH sont insensibles à l'oxydation de l'étape (b),
- X et Y, identiques ou différents, sont choisis parmi un atome d'oxygène, un atome de soufre, une amine de formule -NR₁₁R₁₂, R₁₁ est choisi parmi un atome d'hydrogène, un groupe alkyle, un groupe aryle, substitués ou non, et R₁₂ n'est pas un atome d'hydrogène,
- R₇ à R₁₀, identiques ou différents, sont, soit identiques à quatre au plus des groupes R₁ à R₆, soit, du fait de la transformation d'un ou plusieurs des groupes R₁ à R₆, lors de la réaction de l'étape (a), choisis parmi un atome d'hydrogène, un groupe alkyle substitué ou non, ou un groupe fonctionnel substitué ou non, et au moins un des groupes R₇ à R₁₀ est un groupement Di.

2. Méthode selon la revendication 1, **caractérisée en ce que** l'agent chimique oxydant est choisi parmi H₅IO₆, RuO₂, OsO₄, (CH₃CH₂CH₂)₄N(RuO₄), NaClO₄, NaIO₄, Na₃H₂IO₆, NaMnO₄, K₂OsO₄, KIO₄, KMnO₄, KRuO₄ K₂RuO₄, LiOCl, l'acétate de plomb, le tétrapropyle ammonium periodate, l'acide chromique ou des sels de celui-ci, NaBiO₃, Ph₃BiCO₃, Ca(OCl)₂, les réactifs Ce(IV), Cr(VI), les sels de Co (II), IOAc, I(OAc)₃, N-iodosuccinimide, VO(acac), Pb(OAc)₄, MnO₂, H₂O₂, ou le mélange des réactifs [H₂O₂, Na₂WO₄, H₃PO₄].

3. Méthode selon la revendication 2 **caractérisée en ce que** l'agent chimique oxydant est un sel periodate.

4. Méthode selon l'une des revendications 1, **caractérisée en ce que** R₁ à R₆ sont choisis de façon à ce que la liaison C₁-C₂ fasse partie d'au moins un cycle.

5. Méthode selon la revendication 1, **caractérisée en ce qu'**au moins une paire des groupes R₁ à R₆ forment ensemble un atome d'oxygène, un atome de soufre, ou un groupe de formule -NR₁₁R₁₂ où R₁₁ est choisi parmi: un atome d'hydrogène, un groupe alkyle, un groupe aryle, substitués ou non, et R₁₂ n'est pas un atome d'hydrogène.

6. Méthode selon la revendication 1, **caractérisée en ce qu'**au plus une paire de groupes R₁ à R₆ dont l'un est choisi parmi R₁, et l'autre choisi parmi R₃, R₄ et R₅ forment une double liaison entre les carbones C₁ et C₂.

7. Méthode selon la revendication 1, **caractérisée en ce que** au plus deux paires de groupes R₁ à R₆ dont l'un est choisi parmi R₁, R₂ et R₆ et l'autre choisi respectivement parmi R₃, R₄ et R₅ forment une triple liaison.

8. Méthode selon l'une des revendications 1 à 7, **caractérisée en ce que** les groupes R₁ à R₆ sont sensibles ou insensibles à l'oxydation de l'étape (b) et **en ce que** les étapes (a) et (b) sont non simultanées.

9. Méthode selon l'une des revendications 1 à 7, **caractérisée en ce que** le ou les groupes R₁ à R₆ sont insensibles à l'oxydation de l'étape (b) et **en ce que** les étapes (a) et (b) sont simultanées.

10. Méthode selon l'une des revendications 1 à 9, **caractérisée en ce que** la transformation chimique est une réaction catalytique.

11. Méthode selon la revendication 10, **caractérisée en ce que** la transformation chimique est une réaction enzymatique.

12. Méthode selon l'une quelconque des revendications 1 à 11, **caractérisée en ce que** les groupes R₁ à R₁₀ ou R'₁ à R'₁₀ sont stables dans un milieu réactionnel, choisi parmi un milieu aqueux, un milieu organique ou un milieu biphasique ou en milieu solide.

13. Méthode selon l'une quelconque des revendications 1 à 12, **caractérisée en ce que** l'une ou l'autre ou les deux étapes (a) et (b) sont réalisées dans un milieu réactionnel choisi parmi un milieu aqueux, un milieu organique, un milieu biphasique ou un milieu solide.

14. Méthode selon l'une quelconque des revendications 1 à 13 **caractérisée en ce que** le substrat comprend un ou plusieurs centres chiraux.

15. Méthode selon la revendication 1, **caractérisée en ce que** le substrat répond à la formule(V) suivante : 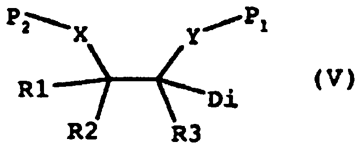 dans laquelle :
- Di, R₁, R₂, R₃, X et Y ont la même signification que dans la revendication 1 et
- Au plus un des groupes P₁ et P₂ est un atome d'hydrogène ou P₁, P₂, identiques ou différents, sont choisis parmi, un groupe acyle substitué par un groupe aryle ou alkyl ou peptidyle, un groupe phosphate, un groupe ester phosphate, un groupe phosphonate, un groupe carbamyle substitué par un groupe aryle ou acyle ou peptidyle, un groupe glycosyle et un groupe sulfate.

16. Méthode selon la revendication 1 ou 4, **caractérisée en ce que** le substrat répond à la formule (VI) suivante : 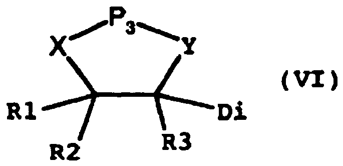 dans laquelle :
- Di R₁, R₂, R₃, X et Y ont la même signification que dans la revendication 1, et
- P₃ est choisi parmi un groupe carbonyle, un groupe -PO₂R₁₁ ou un groupe -OR₁₁PO-, où R₁₁ présente la même signification que dans la revendication 1, un groupe -SO₂, un groupe -CHOR₁₃ où R₁₃ représente un groupe aryle, alkyle ou glycosyle, un groupe SiR₁₄R₁₅ où R₁₄ et R₁₅, identiques ou différents, représentent un groupe aryle, alkyle, aryloxy ou alcoxy et un groupe AsO₂H-.

17. Méthode selon l'une des revendications 1 ou 5, **caractérisée en ce que** le substrat répond à la formule (VII) suivante : 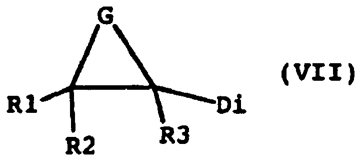 dans laquelle :
- Di, R₁, R₂ et R₃ ont la même signification que dans la revendication 1 et,
- G représente un atome d'oxygène, un atome de soufre ou un groupe amine de formule NR₁₁R₁₂ où R₁₁ et R₁₂ ont la même signification que dans la revendication 1.

18. Méthode selon la revendication 1 ou 6, **caractérisée en ce que** le substrat répond à la formule (VIII) suivante : 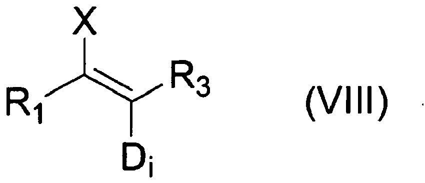 dans laquelle :
- Di, R₁, R₂ et R₃, ont les mêmes significations que dans la revendication 1.

19. Méthode selon la revendication 1, **caractérisée en ce que** le substrat répond à la formule (IX) suivante : 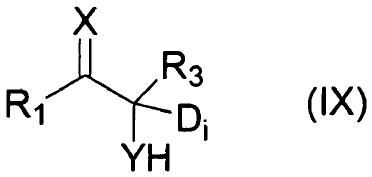 dans laquelle, Di, R₁. R₃, X et Y ont la même signification que dans la revendication 1.

20. Méthode selon l'une quelconque des revendications 1 à 19, **caractérisée en ce que** le produit issu de la transformation de l'étape (a) du substrat répond à la formule (X) suivante : 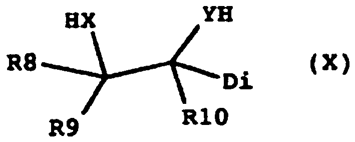 dans laquelle, Di, R₈, R₉, R₁₀, X et Y ont la même signification que dans la revendication 1.

21. Méthode selon l'une des revendications 1 à 20, **caractérisée en ce que** le produit issu de la transformation de l'étape (a) répond à la formule (XV) suivante : 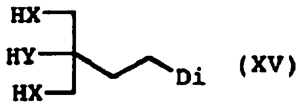 dans laquelle X, Y et Di ont la même signification que dans la revendication 1 et dans laquelle la chaîne portant Di est rattachée à l'une quelconque des positions 1, 2 ou 3 du dérivé glycérolé.

22. Méthode selon l'une quelconque des revendications précédentes **caractérisée en ce que** la réaction d'oxydation est réalisée en milieu organique.

23. Méthode selon l'une quelconque des revendications 1 à 22, **caractérisée en ce que** l'oxydation chimique du composé de formule (II) conduit au produit directement détectable de formule (XI) suivante : 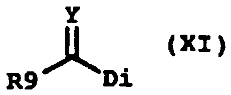 dans laquelle Di, R₉ et Y ont les mêmes significations que dans les revendications précédentes.

24. Méthode selon la revendication 23, **caractérisée en ce que** le produit de formule (XI) est choisi parmi le groupe comprenant une cétone aromatique, un aldéhyde ou une phéromone.

25. Méthode selon la revendication 20 ou 24, **caractérisée en ce que** le groupement Di du produit de la formule (II) ou de la formule (X) répond à la formule (XIII) suivante: 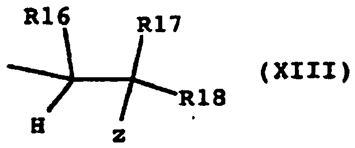 dans laquelle :
- R₁₆, R₁₇ et R₁₈, identiques ou différents, représentent soit un atome d'hydrogène, un groupe alkyle substitué ou non, un groupe fonctionnel substitué ou non,
- Z est un précurseur d'un produit détectable ZH.

26. Méthode selon l'une quelconque des revendications 1 à 25, **caractérisée en ce que** l'oxydation chimique du composé de formule (II), est suivie d'une réaction de béta-élimination et conduit au produit détectable de formule ZH.

27. Méthode selon la revendication 25, **caractérisée en ce que** le produit détectable ZH est choisi parmi un dérivé d'un alcool aromatique, un alcool hétéroaromatique, une amine hétéroaromatique, un atome d'halogène, ou un ester phosphorique.

28. Méthode selon la revendication 26, **caractérisée en ce que** ZH est choisi parmi la fluorescéine, la phenolphtaléine, le rouge de phénol, le p-nitrophénol, l'o-nitrophénol, le 2 , 4-dinitrophénol, l'acide 6-hydroxynaphtoïque, l'acide 8-hydroxy-pyrène 1,3,6-trisulfonique, la tyrosine, la luciférine, le 5-bromo-4-chloro-indolyle, l'indolyle, le quinolinium, le nitro-anilinium, ou la pyridoxamine.

29. Méthode selon l'une des revendications 1 à 22, **caractérisée en ce que** :
- le composé issu de la transformation du substrat après l'étape (a) répond à la formule (XIV) suivante : 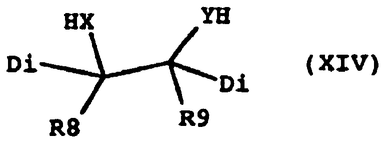 dans laquelle Di, R₈, R₉, X et Y ont la même signification que dans la revendication 1, et **en ce que** :
- il comprend une paire de groupes détectables Di identiques ou différents, l'un attaché au carbone C₁, le deuxième attaché au carbone C₂, et
- les deux groupes détectables Di interagissent entre eux.

30. Méthode selon l'une quelconque des revendications 1 à 29 **caractérisée en ce que** le degré de spécificité du substrat est apporté par la structure de ou des groupes R₁ à R₆ de la formule (I).

31. Méthode de détection d'une transformation chimique d'un substrat, caractérisée en ce l'on soumet ledit substrat à une méthode de libération selon l'une quelconque des revendications 1 à 30 et en ce que l'on détecte le produit libéré.

32. Utilisation d'un composé de formule (V) suivante : 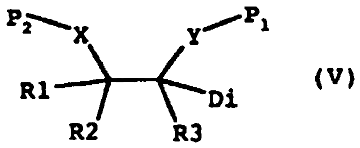 dans laquelle :
- Di, R₁, R₂, R₃, X et Y ont la même signification que dans la revendication 1, et
- au plus un des groupes P₁ et P₂ est un atome d'hydrogène ou P₁, P₂, identiques ou différents, sont choisis parmi, un groupe acyle substitué par un groupe aryle ou alkyl ou peptidyle, un groupe phosphate, un groupe ester phosphate, un groupe phosphonate, un groupe carbamyle substitué par un groupe aryle ou alkyl ou peptidyle, un groupe glycosyle et un groupe sulfate,
en tant que substrat pour la mise en oeuvre d'une méthode selon l'une quelconque des revendications 1, 4 à 15 et 20 à 31.

33. Utilisation d'un composé de formule (VI) suivante : 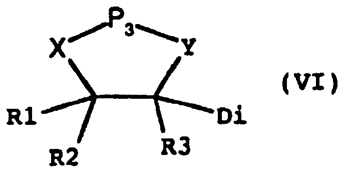 dans laquelle :
- Di R₁, R₂, R₃, X et Y ont la même signification que dans la revendication 1, et
- P₃ est choisi parmi un groupe carbonyle, un groupe -PO₂R₁₁ où un groupe R₁₁PO-, ou R₁₁ présente la même signification que dans la revendication 1, un groupe -SO₂, un groupe -CHOR₁₃ où R₁₃ représente un groupe aryle, alkyle ou glycosyle, un groupe SiR₁₄R₁₅ où R₁₄ et R₁₅, identiques ou différents, représentent un groupe aryle, alkyle, aryloxy ou alcoxy et un groupe AsO₂H-,
en tant que substrat pour la mise en oeuvre d'une méthode selon l'une quelconque des revendications 1, 4 à 14, 16 et 20 à 31.

34. Utilisation d'un composé de formule (VII) suivante : 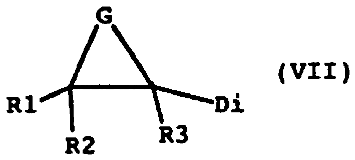 dans laquelle :
- Di, R₁, R₂ et R₃ ont la même signification que dans la revendication 1 et,
- G représente un atome d'oxygène, un atome de soufre ou un groupe amine de formule NR₁₁R₁₂ où R₁₁ R₁₂ ont la même signification que dans la revendication 1,
en tant que substrat pour la mise en oeuvre d'une méthode selon l'une quelconque des revendications 1, 4 à 14, 17 et 20 à 31.

35. Utilisation d'un composé de formule (VIII) suivante : 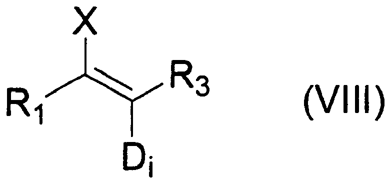 dans laquelle Di, R₁ R₂ et R₃, ont la même signification que dans la revendication 1,
en tant que substrat pour la mise en oeuvre d'une méthode selon l'une quelconque des revendications 1, 4 à 14, 18 et 20 à 21

36. Utilisation d'un composé de formule (IX) suivante : 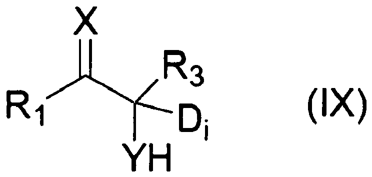 dans laquelle Di, R₁, R₃, X et Y ont la même signification que dans la revendication 1,
en tant que substrat pour la mise en oeuvre d'une méthode selon l'une quelconque des revendications 1, 4 à 14 et 19 à 31.

37. Utilisation selon l'une quelconque des revendications 32 à 36, **caractérisé en ce que** les groupes R₁ à R₃ sont stables dans un milieu réactionnel, notamment aqueux, organique, biphasique, solide.

38. Utilisation d'une méthode de détection d'une transformation chimique selon la revendication 31 pour l'identification de nouveaux catalyseurs, ou de l'activité nouvelle de catalyseurs connus susceptibles de transformer un substrat de formule (I) respectivement en un composé de formule (II).

39. Utilisation d'une méthode de détection d'une transformation chimique selon la revendication 31 pour la détection dudit substrat de formule (I) produit lors d'une réaction chimique.

40. Utilisation selon la revendication 38, **caractérisée en ce que** le catalyseur est contenu dans un échantillon biologique et **en ce que** l'on ajoute audit échantillon un substrat de formule (I).

## Patentansprüche

1. Verfahren zur Detektion der chemischen und/oder enzymatischen Umwandlung eines Substrats in eine detektierbare Verbindung, **dadurch gekennzeichnet, dass** es die folgenden Schritte umfasst:
(a) chemische Umwandlung eines Substrats der Formel (I), worin die C₁-C₂-Bindung gegenüber einer Spaltung durch eine chemische Oxidationsreaktion unempfindlich ist: 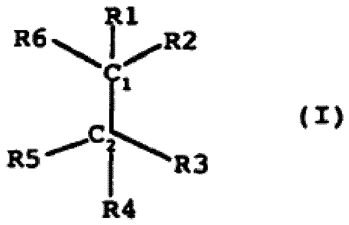 in eine Verbindung der Formel (II), worin die C₁-C₂-Bindung gegenüber einer Spaltung durch eine mit Hilfe eines chemischen Oxidationsmittels durchgeführte chemische Oxidationsreaktion unempfindlich ist: 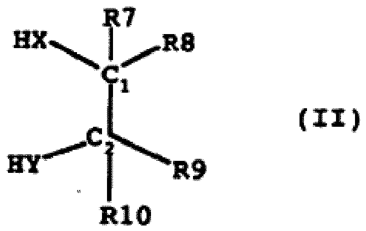 und,
(b) chemische Oxidation der in Schritt (a) erhaltenen Verbindung der Formel (II) unter Spaltung der C₁-C₂-Bindung zum direkten oder indirekten Erhalt eines detektierbaren Produkts,
und dass in den Verbindungen der Formel (I) und (II):
- mindestens eine der Gruppen R₁ bis R₆ eine Gruppierung Di ist, die gegenüber der Oxidationsreaktion von Schritt (b) unempfindlich ist und die nach der Spaltung der C₁-C₂-Bindung detektierbare Eigenschaften aufweist,
- die Gruppen R₁ bis R₆ gleich oder verschieden sind und aus einem Wasserstoffatom, einer gegebenenfalls substituierten Alkylgruppe und einer gegebenenfalls substituierten funktionellen Gruppe ausgewählt sind und im Fall einer funktionellen Gruppe der Formeln -OR₁₂, -SR₁₂ und
- NR₁₁R₁₂, R₁₁ aus einem Wasserstoffatom, einer gegebenenfalls substituierten Alkylgruppe und einer gegebenenfalls substituierten Arylgruppe ausgewählt ist und R₁₂ nicht für ein Wasserstoffatom steht,
- die Gruppe bzw. Gruppen R₁ bis R₆, die nach Schritt (a) die Gruppen der Formel -XH und -YH bilden, gegenüber der Oxidation von Schritt (b) unempfindlich sind,
- X und Y gleich oder verschieden sind und aus einem Sauerstoffatom, einem Schwefelatom und einem Amin der Formel -NR₁₁R₁₂ ausgewählt sind, R₁₁ aus einem Wasserstoffatom, einer gegebenenfalls substituierten Alkylgruppe und einer gegebenenfalls substituierten Arylgruppe ausgewählt ist und R₁₂ nicht für ein Wasserstoffatom steht,
- R₇ bis R₁₀ gleich oder verschieden sind und entweder mit höchstens vier der Gruppen R₁ bis R₆ identisch sind oder infolge der Umwandlung einer oder mehrerer der Gruppen R₁ bis R₆ bei der Reaktion von Schritt (a) aus einem Wasserstoffatom, einer gegebenenfalls substituierten Alkylgruppe oder einer gegebenenfalls substituierten funktionellen Gruppe ausgewählt sind und mindestens eine der Gruppen R₇ bis R₁₀ eine Gruppierung Di ist.

2. Verfahren nach Anspruch 1, **dadurch gekennzeichnet, dass** das chemische Oxidationsmittel aus H₅IO₆, RuO₂, OsO₄, (CH₃CH₂CH₂)₄N(RuO₄), NaClO₄, NaIO₄, Na₃H₂IO₆, NaMnO₄, K₂OsO₄, KIO₄, KMnO₄, KRuO₄, K₂RuO₄, LiOCl, Bleiacetat, Tetrapropylammoniumperiodat, Chromsäure oder Salzen davon, NaBiO₃, Ph₃BiCO₃, Ca(OCl)₂, Ce(IV)- oder Cr(VI)-Reagentien, Co(II)-Salzen, IOA_{C}, I(OAc)₃, N-Iodsuccinimid, VO(acac), Pb(OAc)₄, MnO₂, H₂O₂ oder der Reagentienmischung [H₂O₂, Na₂WO₄, H₃PO₄] ausgewählt wird.

3. Verfahren nach Anspruch 2, **dadurch gekennzeichnet, dass** es sich bei dem chemischen Oxidationsmittel um ein Periodatsalz handelt.

4. Verfahren nach Anspruch 1, **dadurch gekennzeichnet, dass** R₁ bis R₆ so gewählt sind, dass die C₁-C₂-Bindung Teil mindestens eines Rings ist.

5. Verfahren nach Anspruch 1, **dadurch gekennzeichnet, dass** mindestens ein Paar der Gruppen R₁ bis R₆ zusammen ein Sauerstoffatom, ein Schwefelatom oder eine Gruppe der Formel -NR₁₁R₁₂ bilden, wobei R₁₁ aus einem Wasserstoffatom, einer gegebenenfalls substituierten Alkylgruppe und einer gegebenenfalls substituierten Arylgruppe ausgewählt ist und R₁₂ nicht für ein Wasserstoffatom steht.

6. Verfahren nach Anspruch 1, **dadurch gekennzeichnet, dass** höchstens ein Paar der Gruppen R₁ bis R₆, von denen eine aus R₁ ausgewählt ist und die andere aus R₃, R₄ und R₅ ausgewählt ist, eine Doppelbindung zwischen den Kohlenstoffatomen C₁ und C₂ bildet.

7. Verfahren nach Anspruch 1, **dadurch gekennzeichnet, dass** höchstens zwei Paare der Gruppen R₁ bis R₆, von denen eine aus R₁, R₂ und R₆ ausgewählt ist und die andere aus R₃, R₄ und R₅ ausgewählt ist, eine Dreifachbindung bilden.

8. Verfahren nach einem der Ansprüche 1 bis 7, **dadurch gekennzeichnet, dass** die Gruppen R₁ bis R₆ gegenüber der Oxidation von Schritt (b) empfindlich oder unempfindlich sind und dass die Schritte (a) und (b) nicht gleichzeitig sind.

9. Verfahren nach einem der Ansprüche 1 bis 7, **dadurch gekennzeichnet, dass** die Gruppe bzw. Gruppen R₁ bis R₆ gegenüber der Oxidation von Schritt (b) unempfindlich sind und dass die Schritte (a) und (b) gleichzeitig sind.

10. Verfahren nach einem der Ansprüche 1 bis 9, **dadurch gekennzeichnet, dass** es sich bei der chemischen Umwandlung um eine katalytische Reaktion handelt.

11. Verfahren nach Anspruch 10, **dadurch gekennzeichnet, dass** es sich bei der chemischen Umwandlung um eine enzymatische Reaktion handelt.

12. Verfahren nach einem der Ansprüche 1 bis 11, **dadurch gekennzeichnet, dass** die Gruppen R₁ bis R₁₀ oder R'₁ bis R'₁₀ in einem aus einem wässrigen Medium, einem organischen Medium oder einem zweiphasigen Medium oder einem festen Medium ausgewählten Reaktionsmedium stabil sind.

13. Verfahren nach einem der Ansprüche 1 bis 12, **dadurch gekennzeichnet, dass** man Schritt (a) und/oder (b) in einem aus einem wässrigen Medium, einem organischen Medium oder einem zweiphasigen Medium oder einem festen Medium ausgewählten Reaktionsmedium durchführt.

14. Verfahren nach einem der Ansprüche 1 bis 13, **dadurch gekennzeichnet, dass** das Substrat ein oder mehrere Chiralitätszentren umfasst.

15. Verfahren nach Anspruch 1, **dadurch gekennzeichnet, dass** das Substrat der folgenden Formel (V) entspricht: 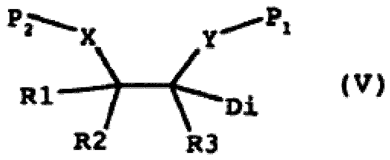 worin:
- Di, R₁, R₂, R₃, X und Y die gleiche Bedeutung wie in Anspruch 1 besitzen und
- höchstens eine der Gruppen P₁ und P₂ ein Wasserstoffatom ist oder P₁ und P₂ gleich oder verschieden sind und aus einer Acylgruppe, die durch eine Aryl- oder Alkyl- oder Peptidylgruppe substituiert ist, einer Phosphatgruppe, einer Phosphatestergruppe, einer Phosphonatgruppe, einer Carbamylgruppe, die durch eine Aryl- oder Acyl-oder Peptidylgruppe substituiert ist, einer Glykosylgruppe und einer Sulfatgruppe ausgewählt sind.

16. Verfahren nach Anspruch 1 oder 4, **dadurch gekennzeichnet, dass** das Substrat der folgenden Formel (VI) entspricht: 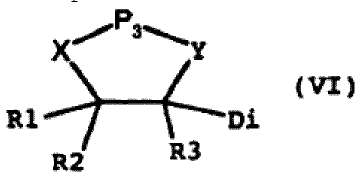 worin:
- Di, R₁, R₂, R₃, X und Y die gleiche Bedeutung wie in Anspruch 1 besitzen und
- P₃ aus einer Carbonylgruppe, einer -PO₂R₁₁-Gruppe oder einer -OR₁₁PO-Gruppe, wobei R₁₁ die gleiche Bedeutung wie in Anspruch 1 besitzt, einer -SO₂-Gruppe, einer -CHOR₁₃-Gruppe, wobei R₁₃ für eine Aryl-, Alkyl- oder Glykosylgruppe steht, einer SiR₁₄R₁₅-Gruppe, wobei R₁₄ und R₁₅ gleich oder verschieden sind und für eine Aryl-, Alkyl-, Aryloxy- oder Alkoxygruppe stehen, und einer AsO₂H-Gruppe ausgewählt ist.

17. Verfahren nach einem der Ansprüche 1 oder 5, **dadurch gekennzeichnet, dass** das Substrat der folgenden Formel (VII) entspricht: 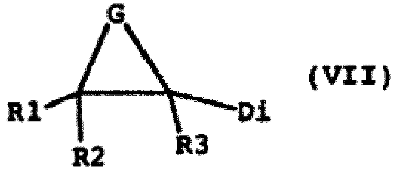 worin:
- Di, R₁, R₂ und R₃ die gleiche Bedeutung wie in Anspruch 1 besitzen und
- G für ein Sauerstoffatom, ein Schwefelatom oder eine Amingruppe der Formel NR₁₁R₁₂ steht, wobei R₁₁ und R₁₂ die gleiche Bedeutung wie in Anspruch 1 besitzen.

18. Verfahren nach Anspruch 1 oder 6, **dadurch gekennzeichnet, dass** das Produkt der folgenden Formel (VIII) entspricht: 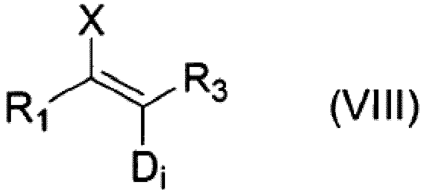 worin:
- Di, R₁, R₂ und R₃ die gleiche Bedeutung wie in Anspruch 1 besitzen.

19. Verfahren nach Anspruch 1, **dadurch gekennzeichnet, dass** das Produkt der folgenden Formel (IX) entspricht: 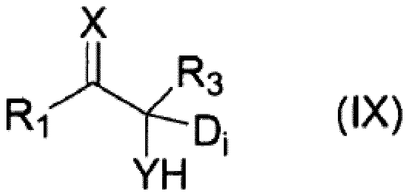 worin:
- Di, R₁, R₃, X und Y die gleiche Bedeutung wie in Anspruch 1 besitzen.

20. Verfahren nach einem der Ansprüche 1 bis 19, **dadurch gekennzeichnet, dass** das Produkt der Substratumwandlung von Schritt (a) der folgenden Formel (X) entspricht: 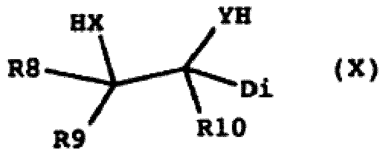 worin:
- Di, R₈, R₉, R₁₀, X und Y die gleiche Bedeutung wie in Anspruch 1 besitzen.

21. Verfahren nach einem der Ansprüche 1 bis 20, **dadurch gekennzeichnet, dass** das Produkt der Umwandlung von Schritt (a) der folgenden Formel (XV) entspricht: 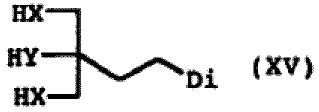 worin X, Y und Di die gleiche Bedeutung wie in Anspruch 1 besitzen und die Di tragende Kette an eine beliebige der Positionen 1, 2 oder 3 des Glycerinderivats gebunden ist.

22. Verfahren nach einem der vorhergehenden Ansprüche, **dadurch gekennzeichnet, dass** die Oxidationsreaktion in einem organischen Medium durchgeführt wird.

23. Verfahren nach einem der Ansprüche 1 bis 22, **dadurch gekennzeichnet, dass** die chemische Oxidation der Verbindung der Formel (II) zu dem direkt detektierbaren Produkt der folgenden Formel (XI) führt: 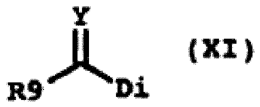 worin Di, R₉ und Y die in den vorhergehenden Ansprüchen angegebenen Bedeutungen besitzen.

24. Verfahren nach Anspruch 23, **dadurch gekennzeichnet, dass** das Produkt der Formel (XI) aus der ein aromatisches Keton, einen Aldehyd oder ein Pheromon umfassenden Gruppe ausgewählt wird.

25. Verfahren nach Anspruch 20 oder 24, **dadurch gekennzeichnet, dass** die Gruppierung Di das Produkt der Formel (II) bzw. der Formel (X) der folgenden Formel (XIII) entspricht: 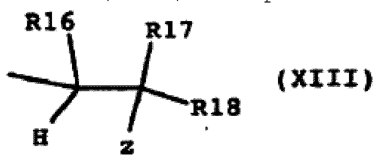 worin:
- R₁₆, R₁₇ und R₁₈ gleich oder verschieden sind und für ein Wasserstoffatom, eine gegebenenfalls substituierte Alkylgruppe oder eine gegebenenfalls substituierte funktionelle Gruppe stehen,
- Z eine Vorstufe eines detektierbaren Produkts ZH ist.

26. Verfahren nach einem der Ansprüche 1 bis 25, **dadurch gekennzeichnet, dass** die chemische Oxidation der Verbindungformel (II) von einer Beta-Eliminierungsreaktion gefolgt wird und zu dem detektierbaren Produkt der Formel ZH führt.

27. Verfahren nach Anspruch 25, **dadurch gekennzeichnet, dass** das detektierbare Produkt ZH aus einem Derivat eines aromatischen Alkohols, einem heteroaromatischen Alkohol, einem heteroaromatischen Amin, einem Halogenatom oder einem Phosphorsäureester ausgewählt wird.

28. Verfahren nach Anspruch 26, **dadurch gekennzeichnet, dass** ZH aus Fluoreszein, Phenolphthalein, Phenolrot, p-Nitrophenol, o-Nitrophenol, 2,4-Dinitrophenol, 6-Hydroxynaphthoesäure, 8-Hydroxypyren-1,3,6-trisulfonsäure, Tyrosin, Luciferin, 5-Brom-4-chlorindolyl, Indolyl, Chinolinium, Nitroanilinium oder Pyridoxamin ausgewählt wird.

29. Verfahren nach einem der Ansprüche 1 bis 22, **dadurch gekennzeichnet, dass**:
- die Verwendung aus der Substratsumwandlung nach Schritt (a) der folgenden Formel (XIV) entspricht: 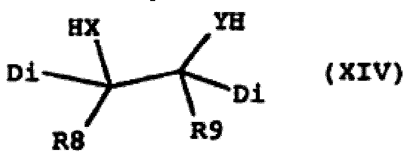 worin Di, R₈, R₉, X und Y die gleiche Bedeutung wie in Anspruch 1 besitzen, und dass:
- sie ein Paar von gleichen oder verschiedenen detektierbaren Gruppen Di enthalten, von denen die eine an den Kohlenstoff C₁ gebunden ist und die andere an den Kohlenstoff C₂ gebunden ist, und
- die beiden detektierbaren Gruppen Di miteinander wechselwirken.

30. Verfahren nach einem der Ansprüche 1 bis 29, **dadurch gekennzeichnet, dass** der Spezifitätsgrad des Substrats durch die Struktur der Gruppe bzw.
Gruppen R₁ bis R₆ der Formel (I) bereitgestellt wird.

31. Verfahren zur Detektion einer chemischen Umwandlung eines Substrats, **dadurch gekennzeichnet, dass** man das Substrat einem Freisetzungsverfahren nach einem der Ansprüche 1 bis 30 unterwirft und das freigesetzte Produkt detektiert.

32. Verwendung einer Verbindung der folgenden Formel (V): 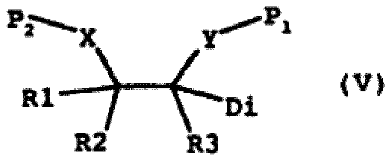 worin:
- Di, R₁, R₂, R₃, X und Y die gleiche Bedeutung wie in Anspruch 1 besitzen und
- höchstens eine der Gruppen P₁ und P₂ ein Wasserstoffatom ist oder P₁ und P₂ gleich oder verschieden sind und aus einer Acylgruppe, die durch eine Aryl- oder Alkyl- oder Peptidylgruppe substituiert ist, einer Phosphatgruppe, einer Phosphatestergruppe, einer Phosphonatgruppe, einer Carbamylgruppe, die durch eine Aryl- oder Alkyl-oder Peptidylgruppe substituiert ist, einer Glykosylgruppe und einer Sulfatgruppe ausgewählt sind,
als Substrat zur Durchführung eines Verfahrens nach einem der Ansprüche 1, 4 bis 15 und 20 bis 31.

33. Verwendung einer Verbindung der folgenden Formel (VI): 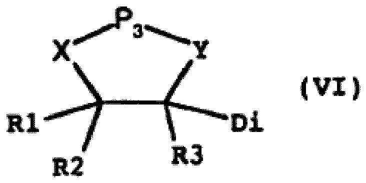 worin:
- Di, R₁, R₂, R₃, X und Y die gleiche Bedeutung wie in Anspruch 1 besitzen und
- P₃ aus einer Carbonylgruppe, einer -PO₂R₁₁-Gruppe oder einer R₁₁PO-Gruppe, wobei R₁₁ die gleiche Bedeutung wie in Anspruch 1 besitzt, einer -SO₂-Gruppe, einer -CHOR₁₃-Gruppe, wobei R₁₃ für eine Aryl-, Alkyl- oder Glykosylgruppe steht, einer SiR₁₄R₁₅-Gruppe, wobei R₁₄ und R₁₅ gleich oder verschieden sind und für eine Aryl-, Alkyl-, Aryloxy- oder Alkoxygruppe stehen, und einer AsO₂H-Gruppe ausgewählt ist,
als Substrat zur Durchführung eines Verfahrens nach einem der Ansprüche 1, 4 bis 14, 16 und 20 bis 31.

34. Verwendung einer Verbindung der folgenden Formel (VII): 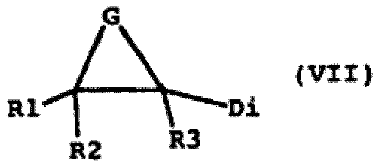 worin:
- Di, R₁, R₂ und R₃ die gleiche Bedeutung wie in Anspruch 1 besitzen und
- G für ein Sauerstoffatom, ein Schwefelatom oder eine Amingruppe der Formel NR₁₁R₁₂ steht, wobei R₁₁ und R₁₂ die gleiche Bedeutung wie in Anspruch 1 besitzen,
als Substrat zur Durchführung eines Verfahrens nach einem der Ansprüche 1, 4 bis 14, 17 und 20 bis 31.

35. Verwendung einer Verbindung der folgenden Formel (VIII): 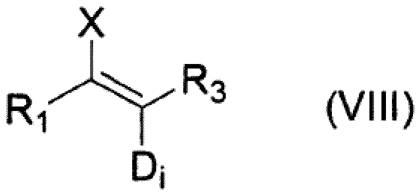 worin Di, R₁, R₂ und R₃ die gleiche Bedeutung wie in Anspruch 1 besitzen,
als Substrat zur Durchführung eines Verfahrens nach einem der Ansprüche 1, 4 bis 14, 18 und 20 bis 21.

36. Verwendung einer Verbindung der folgenden Formel (IX) : 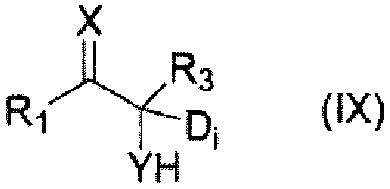 worin Di, R₁, R₃ und X und Y die gleiche Bedeutung wie in Anspruch 1 besitzen,
als Substrat zur Durchführung eines Verfahrens nach einem der Ansprüche 1, 4 bis 14 und 19 bis 31.

37. Verwendung nach einem der Ansprüche 32 bis 36, **dadurch gekennzeichnet, dass** die Gruppen R₁ bis R₃ in einem Reaktionsmedium, insebsondere einem wässrigen, organischen, zweiphasigen oder festen Reaktionsmedium, stabil sind.

38. Verwendung eines Verfahrens zur Detektion einer chemischen Umwandlung nach Anspruch 31 zur Identifizierung von neuen Katalysatoren oder der neuen Aktivität bekannter Katalysatoren, die ein Substrat der Formel (I) respektive in eine Verbindung der Formel (II) umwandeln können.

39. Verwendung eines Verfahrens zur Detektion einer chemischen Umwandlung nach Anspruch 31 zur Detektion des bei einer chemischen Reaktion gebildeten Substrats der Formel (I).

40. Verwendung nach Anspruch 38, **dadurch gekennzeichnet, dass** der Katalysator in einer biologischen Probe enthalten ist und dass man der Probe ein Substrat der Formel (I) zusetzt.

## Claims

1. Method for detecting the chemical and/or enzymatic conversion of a substrate into a detectable compound, **characterized in that** it comprises the following steps:
a) the chemical conversion of a substrate of formula (I) wherein the C₁-C₂ bond is insensitive to cleavage by chemical oxidation reaction: 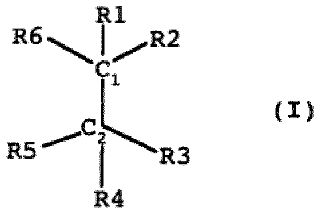 into a compound of formula (II) wherein the C₁-C₂ bond is sensitive to cleavage by a chemical oxidation reaction carried out by means of a chemical oxidizing agent: 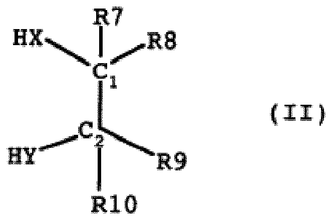 and,
(b) the chemical oxidization of the compound of chemical formula (II) obtained in step (a) which split the C₁-C₂ bond to obtain directly or indirectly detectable product,
and **in that** in the compounds of formulas (I) and (II):
- at least one of R₁ to R₆ groups is a Di group which is insensitive to the oxidation reaction of step (b) and having detectable properties after the cleavage of the C₁-C₂ bond,
- R₁ à R₆ groups, identical or different, are chosen from a hydrogen atom, a substituted or unsubstituted alkyl group, a substituted or unsubstituted functional group and in the case of a functional group of the formulas -OR₁₂, -SR₁₂, -NR₁₁R₁₂, R₁₁ is selected from a hydrogen atom, un alkyl group, an aryl group, substituted or unsubstituted, and R₁₂ is not a hydrogen atom,
- the group or R₁ to R₆ groups forming after step (a) groups of formula -XH and -YH are insensitive to the oxidation of step (b),
- X and Y, identical or different, are selected from an oxygen atom, a sulfur atom, an amine of the formula -NR₁₁R₁₂, R₁₁ is selected from a hydrogen atom, un alkyl group, an aryl group, substituted or unsubstituted, and R₁₂ is not a hydrogen atom,
- R₇ to R₁₀, identical or different, are either identical to at most four of the R₁ to R₆ groups, or, because of the transformation of one or more of R₁ to R₆ groups, during the reaction of step (a), selected from a hydrogen atom, a substituted or unsubstituted alkyl group, or a substituted or unsubstituted functional group, and at least one of R₇ to R₁₀ groups is a group Di.

2. Method according to claim 1, **characterized in that** the chemical oxidizing agent is selected from H₅IO₆, RuO₂, OsO₄, (CH₃CH₂CH₂)₄N(RuO₄), NaClO₄, NalO₄, Na₃H₂IO₆, NaMnO₄, K₂OsO₄, KIO₄, KMnO₄, KRuO₄, K₂RuO₄, LiOCl, lead acetate, the tetrapropyl ammonium periodate, chromic acid or salts thereof, NaBiO₃, Ph₃BiCO₃, Ca(OCl)₂, the reagents Ce(IV), Cr(VI), the salts of Co (II), IOAc, I(OAc)₃, N-iodosuccinimide, VO(acac), Pb(OAc)₄, MnO₂, H₂O₂, or the mixture of reactants [H₂O₂, Na₂WO₄, H₃PO₄].

3. Method according to claim 2 **characterized in that** the chemical oxidizing agent is a periodate salt.

4. Method according to one of Claims 1, **characterized in that** R₁ to R₆ are chosen so that the C₁-C₂ bond is part of at least one ring.

5. Method according to claim 1, **characterized in that** at least one pair of R₁ to R₆ groups forms together an oxygen atom, a sulfur atom, or a group of formula -NR₁₁R₁₂ wherein R₁₁ is selected from: an hydrogen atom, an alkyl group, an aryl group, substituted or unsubstituted, and R₁₂ is not an hydrogen atom.

6. Method according to claim 1, **characterized in that** at most a pair of groups which R₁ to R₆ groups among which one is selected from R₁, and the other is selected from R₃, R₄ and R₅ form a double bond between carbons C₁ and C₂.

7. Method according to claim 1, **characterized in that** at most than two pairs of R₁ to R₆ groups among which one is selected from R₁, R₂ and R₆ and the other is respectively selected from R₃, R₄ and R₅ form a triple bond.

8. Method according to one of claims 1 to 7, **characterized in that** the R₁ to R₆ groups are sensitive or insensitive to the oxidation of step (b) and **in that** steps (a) and (b) are not simultaneous.

9. Method according to one of claims 1 to 7, **characterized in that** the R₁ to R₆ groups are insensitive to the oxidation of step (b) and **in that** steps (a) and (b) are simultaneous

10. Method according to one of claims 1 to 9, **characterized in that** the chemical conversion is a catalytic reaction.

11. Method according to claim 10, **characterized in that** the chemical conversion is an enzymatic reaction.

12. Method according to any one of claims 1 to 11, **characterized in that** the R₁ to R₁₀ groups or R'₁ to R'₁₀ groups are stable in a reaction medium selected from an aqueous medium, an organic medium or a biphasic medium or solid medium.

13. Method according to any one of claims 1 to 12, **characterized in that** one or the other or both of steps (a) and (b) are performed in a reaction medium selected from an aqueous medium, an organic medium, a biphasic medium or a solid medium.

14. Method according to any one of claims 1 to 13 **characterized in that** the substrate comprises one or more chiral centers.

15. Method according to claim 1, **characterized in that** the substrate has the following formula (V): 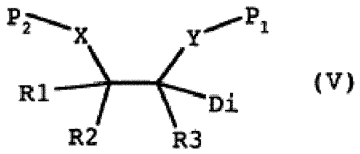 wherein:
- Di, R₁, R₂, R₃, X and Y have the same meaning as in claim 1 and
- At most one of the P₁ and P₂ groups is a hydrogen atom or P₁, P₂, identical or different, are selected from an acyl group substituted by an aryl or alkyl or peptidyl group, a phosphate group, a phosphate ester group, a phosphonate group, a carbamoyl group substituted with aryl or acyl or peptidyl group, a glycosyl group and a sulfate group.

16. Method according to claim 1 or 4, **characterized in that** the substrate has the following formula (VI): 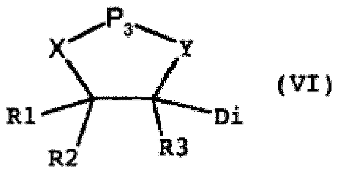 wherein:
- Di, R₁, R₂, R₃, X and Y have the same meaning as in claim 1, and
- P₃ is selected from a carbonyl group, a -PO₂R₁₁ group or a - OR₁₁PO- group, wherein R₁₁ has the same meaning as in claim 1, a -SO₂ group, a -CHOR₁₃ group wherein R₁₃ is aryl, alkyl or glycosyl group, a SiR₁₄R₁₅ group where R₁₄ and R₁₅, identical or different, represent an aryl, an alkyl, an aryloxy or an alkoxy and a AsO₂H- group.

17. Method according to one of claims 1 or 5, **characterized in that** the substrate has the following formula (VII): 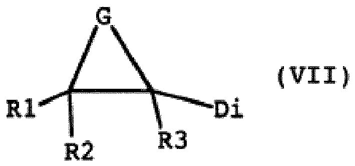 wherein:
- Di, R₁, R₂, R₃, X and Y have the same meaning as in claim 1, and
- G represents an oxygen atom, a sulfur atom or an amine group of formula NR₁₁R₁₂ wherein R₁₁ and R₁₂ have the same meaning as in claim 1.

18. Method according to claim 1 or 6, **characterized in that** the substrate has the following formula (VIII): 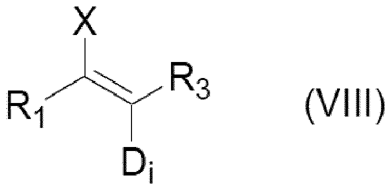 wherein:
- Di, R₁, R₂, R₃, X and Y have the same meaning as in claim 1.

19. Method according to claim 1, **characterized in that** the substrate has the following formula (IX): 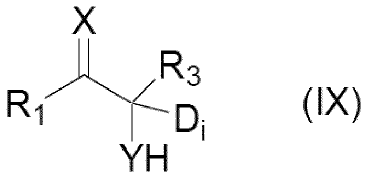 wherein Di, R₁, R₃, X and Y have the same meaning as in claim 1.

20. Method according to any one of claims 1 to 19, **characterized in that** the product resulting from the conversion of step (a) of the substrate has the following formula (X): 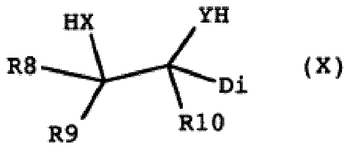 wherein Di, R₈, R₉, R₁₀, X and Y have the same meaning as in claim 1.

21. Method according to one of claims 1 to 20, **characterized in that** the product resulting from the conversion of step (a) has the following formula (XV): 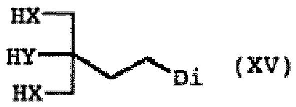 wherein X, Y and Di have the same meaning as in claim 1 and wherein the chain bearing Di is connected to any one of positions 1, 2 or 3 of the glycerol derivative.

22. Method according to any one of the preceding claims **characterized in that** the oxidation reaction is carried out in an organic medium.

23. Method according to any one of claims 1 to 22, **characterized in that** the chemical oxidation of the compound of formula (II) leads to a product directly detectable having the following formula (XI): 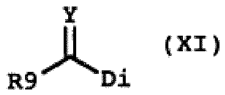 wherein Di, R₉ and Y have the same meanings as in the preceding claims.

24. Method according to claim 23, **characterized in that** the product of formula (XI) is selected from the group comprising of an aromatic ketone, an aldehyde or a pheromone.

25. Method according to claim 20 or 24, **characterized in that** the Di group from product of formula (II) or formula (X) has the following formula (XIII): 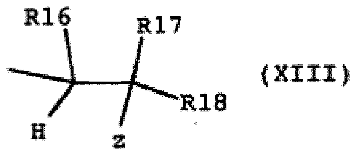 wherein:
- R₁₆, R₁₇ and R₁₈, identical or different, represent either a hydrogen atom, a substituted or unsubstituted alkyl group, a substituted or unsubstituted functional group,
- Z is a precursor of a detectable product ZH.

26. Method according to any one of claims 1 to 25, **characterized in that** the chemical oxidation of the compound of formula (II), is followed by a beta-elimination reaction and leads to the detectable product of the formula ZH.

27. Method according to claim 25, **characterized in that** the detectable product ZH is selected from a derivative of an aromatic alcohol, a heteroaromatic alcohol, a heteroaromatic amine, a halogen atom, or a phosphoric ester.

28. Method according to claim 26, **characterized in that** ZH is selected from fluorescein, phenolphthalein, phenol red, p-nitrophenol, o-nitrophenol, 2,4-dinitrophenol, 6-hydroxynaphthoic acid, 8-hydroxy-pyrene 1,3,6-trisulfonic acid, tyrosine, luciferin, 5-bromo-4-chloro-indolyl, indolyl, quinolinium, nitro-aniline, or pyridoxamine.

29. Method according to one of claims 1 to 22, **characterized in that**:
- the compound derived from the conversion of the substrate after step (a) has the following formula (XIV): 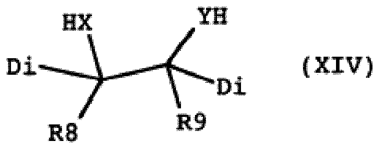 wherein Di, R₈, R₉, X and Y have the same meaning as in claim 1, and **in that**:
- it comprises a pair of detectable groups Di identical or different, one attached to the carbon C₁, the second attached to the carbon C₂, and
- the two groups detectable Di interact amongst themselves.

30. Method according to any one of claims 1 to 29 **characterized in that** the degree of specificity of the substrate is provided by the structure of the group or groups R₁ to R₆ of formula (I).

31. Method for detecting a chemical conversion of a substrate, **characterized in that** said substrate is subjected to a release method according to any one of claims 1 to 30 and **in that** the released substance is detected.

32. Use of a compound of the following formula (V): 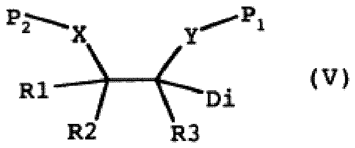 wherein:
- Di, R₁, R₂, R₃, X and Y have the same meaning as in claim 1, and
- at most one of the groups P₁ and P₂ is hydrogen or P₁, P₂, identical or different, are selected from an acyl group substituted by an aryl or alkyl or peptidyl group, a phosphate group, a phosphate ester, a phosphonate group, a carbamoyl group substituted with an aryl or alkyl or peptidyl group, a glycosyl group and a sulfate group,
as a substrate for the implementation of a method according to any one of claim 1, 4 to 15 and 20 to 31.

33. Use of a compound of the following formula (VI): 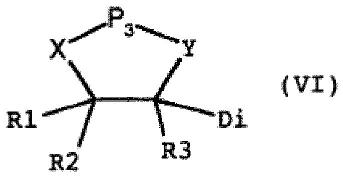 wherein:
- Di R₁, R₂, R₃, X and Y have the same meaning as in claim 1, and
- P₃ is selected from a carbonyl group, a -PO₂R₁₁ group or a R₁₁PO- group, wherein R₁₁ has the same meaning as in claim 1, a -SO₂ group, an -CHOR₁₃ group wherein R₁₃ is an aryl, alkyl or glycosyl group, a SiR₁₄R₁₅ group wherein R₁₄ and R₁₅, identical or different, represent an aryl, alkyl, aryloxy or alkoxy group and an AsO₂H- group,
as a substrate for the implementation of a method according to any one of claims 1, 4 to 14, 16 and 20 to 31.

34. Use of a compound of the following formula (VII): 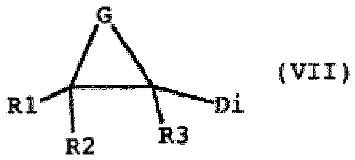 wherein:
- Di, R₁, R₂ and R₃ have the same meaning as in claim 1 and,
- G represents an oxygen atom, a sulfur atom or a amine group of formula NR₁₁R₁₂ wherein R₁₁ R₁₂ have the same meaning as in claim 1,
as a substrate for the implementation of a method according to any one of claims 1, 4 to 14, 17 and 20 to 31.

35. Use of a compound of the following formula (VIII): 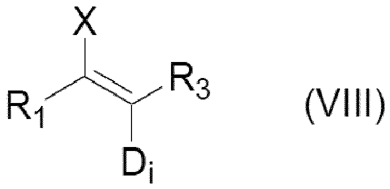 wherein Di, R₁, R₂ and R₃ have the same meaning as in claim 1,
as a substrate for the implementation of a method according to any one of claims 1, 4 to 14, 18 and 20 to 21

36. Use of a compound of the following formula (IX): 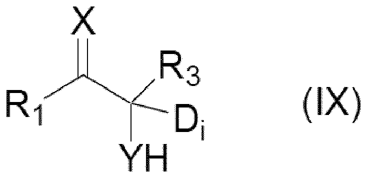 wherein Di, R₁, R₃, X and Y have the same meaning as in claim 1,
as a substrate for the implementation of a method according to any one of claims 1, 4 to 14 and 19 to 31.

37. Use according to any one of claims 32 to 36, **characterized in that** the groups R₁ to R₃ are stable in the reaction medium, especially aqueous, organic, biphasic, solid.

38. Use of a method for detecting a chemical conversion according to claim 31 for identifying novel catalysts, or new activity of known catalysts capable of transforming a substrate of formula (I) respectively into a compound of formula (II).

39. Use of a method for detecting a chemical conversion according to claim 31 for the of said substrate of formula (I) produced during a chemical reaction.

40. Use according to claim 38, **characterized in that** the catalyst is contained in a biological sample and **in that** a substrate of formula (I) is added to said sample.
